(19) Europäisches Patentamt
European Patent Office
Office européen des brevets

(11) Publication number: 0 184 322 B1

(12) **EUROPEAN PATENT SPECIFICATION**

(45) Date of publication of the patent specification: **20.12.89**

(51) Int. Cl.⁴: **C07D 401/12**, A61K 31/415, A61K 31/44, C07D 491/04, C07D 213/74, C07D 213/73

(21) Application number: **85307928.3**

(22) Date of filing: **31.10.85**

(54) **Benzimidazoles.**

(30) Priority: **02.11.84 GB 8427836**
**21.12.84 GB 8432515**
**17.07.85 GB 8518043**

(43) Date of publication of application:
**11.06.86 Bulletin 86/24**

(45) Publication of the grant of the patent:
**20.12.89 Bulletin 89/51**

(84) Designated Contracting States:
**AT BE CH DE FR GB IT LI LU NL SE**

(56) References cited:
**EP-A- 0 074 341**
**EP-A- 0 150 586**
**EP-A- 0 174 717**
**EP-A- 0 176 308**
**EP-A- 0 178 438**
**EP-A- 0 806 002**
**WO-A-86/02645**

**The file contains technical information submitted after the application was filed and not included in this specification**

(73) Proprietor: **SMITH KLINE & FRENCH LABORATORIES LIMITED, Mundells, Welwyn Garden City Hertfordshire, AL7 1EY(GB)**

(72) Inventor: **Ife, Robert John, 19 Mobbsbury Way, Stevenage Hertfordshire(GB)**

(74) Representative: **Waters, David Martin, Dr. et al, Smith Kline & French Laboratories Ltd. Patent Department Mundells, Welwyn Garden City Hertfordshire AL7 1EY(GB)**

EP 0 184 322 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to novel substituted benzimidazole derivatives, processes for their preparation, intermediates useful in their preparation, pharmaceutical compositions containing them and their use in therapy.

Substituted benzimidazole derivatives that are capable of inhibiting gastric acid secretion are known in the art. For example, GB 1 500 043 and GB 1 525 958 disclose a series of 2-pyridylalkylthio- and 2-pyridylalkylsulphinyl benzimidazoles in which the pyridyl group is optionally mono-substituted by an alkyl or a halogen group. Further, EP 5 129B, 74 341A and 80 602A disclose series of 2-pyridylalkylsulphinyl- and 2-pyridylalkylthio-benzimidazoles in which the pyridyl group is optionally substituted by 1 to 3 substituents selected from methyl, ethyl, methoxy, ethoxy, methoxyethoxy or ethoxyethoxy; and GB 2 134 523A discloses further such compounds in which the pyridyl group is optionally substituted by 1 to 3 groups.

In addition, a number of co-pending European Patent Applications which form part of the state of the art according to Article 54(3) EPC have been published which disclose 2-pyridylalkylsulphinyl benzimidazole compounds in which the pyridyl ring is substituted by alternative substituents, in particular, EP 150 586A discloses such compounds in which the pyridine ring is substituted by inter alia, a 4-N-heterocyclyc group or an alkylthio group; PCT(EP) WO 86/02 645-A discloses such compounds in which the pyridyl group is substituted by a 4-amino group; EP 174 717A discloses a wide range of compounds containing substituted pyridyl rings and also aminophenyl rings in place of said pyridyl rings; EP 178 438A discloses such compounds in which the pyridyl ring can be substituted by a 4-amino substituent and also compounds in which the pyridyl ring is replaced by a pyrimidinyl ring, and EP 176 358 discloses further compounds in which the pyridyl ring is substituted by a 4-amino substituent. There is no specific disclosure however in any of the foregoing of compounds in which the pyridyl ring is substituted by both an amino and a halogen moiety. Such compounds are believed to exert their effects by inhibition of the gastro-intestinal $H^+$–$K^+$ ATPase enzyme (Fellenius E., Berglindh, T., Sachs, G., Olke, L., Elander, B., Sjostrand, S.E. and Wallmark B., 1981, Nature, 290, 159–61).

In addition, US 4 359 465 discloses the cytoprotective action of certain 2-pyridylalkylthio- and 2-pyridylalkylsulphinyl benzimidazoles and their use in the treatment or prevention of gastro-intestinal inflammatory disease.

The compounds of the present invention are 2-pyridylalkylsulphinyl- and 2-pyridylalkylthio-benzimidazoles in which the 2-pyridyl group is substituted in the 4-position by an optionally substituted amino group and in the 3- or 5-positions by a halogen atom. The compounds are inhibitors of potassium stimulated $H^+$–$K^+$ ATPase activity.

The present invention therefore provides, in a first aspect, a compound of structure (I)

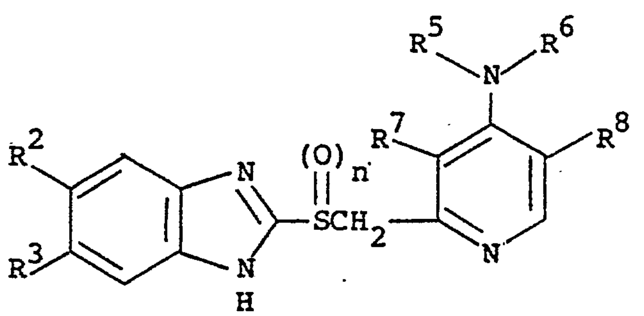

(I)

in which,

$R^2$ and $R^3$ are the same or different and are each hydrogen, $C_{1-6}$alkyl, $C_{1-6}$alkoxy, $RCF_2O$, an ethoxy group substituted by 3 to 5 fluorine atoms, or $R^2$ and $R^3$ together form a group $-O(CR_2)_mO-$;

R is hydrogen or fluorine;

m is 1 or 2;

n is 0 or 1;

$R^5$ and $R^6$ are the same or different and are each $C_{1-6}$alkyl or $R^5$ and $R^6$ together with the nitrogen atom to which they are attached form a piperidino, or morpholino group; and

one of $R^7$ and $R^8$ is halogen, and the other is hydrogen, halogen or $C_{1-6}$alkyl;

or a pharmaceutically acceptable salt thereof.

Suitably, $R^2$ and $R^3$ are both hydrogen. Preferably one of $R^2$ and $R^3$ is hydrogen and the other is $C_{1-6}$alkyl. More preferably, one of $R^2$ and $R^3$ is hydrogen and the other is $C_{1-6}$alkoxy, $RCF_2O$ or an ethoxy group substituted by 3 to 5 fluorine atoms; or, $R^2$ and $R^3$ together form a group $-O(CR_2)_mO-$.

Suitably, ethoxy groups substituted by 3 to 5 fluorine atoms are, 2,2,2-trifluoroethoxy, 1,1,2-trifluoroethoxy 1,2,2-trifluoroethoxy, 1,2,2,2-tetrafluoroethoxy and perfluoroethoxy. Preferably, ethoxy groups substituted by 3 to 5 fluorine atoms are 1,1,2,2-tetrafluoroethoxy.

Suitably, groups $-O(CR_2)_mO-$ in which m is 1 are methylenedioxy ($-OCH_2O-$); preferably difluorome-

thylenedioxy ($-OCF_2O-$). Suitably, groups $-O(CR_2)_mO-$ in which m is 2 are ethylenedioxy ($-OCH_2$-$CH_2O-$); preferably, trifluoroethylenedioxy ($-OCHFCF_2O-$).

Suitably n is 0. Preferably n is 1.

Preferably $R^5$ and $R^6$ are the same or different and are each $C_{1-6}$alkyl.

Preferably, $R^5$ and $R^6$ together with the nitrogen to which they are attached form a morpholino or a piperidino group.

Suitable $R^7$ and $R^8$ are both halogen.

Preferably, one of $R^7$ and $R^8$ is halogen and the other is hydrogen or $C_{1-6}$alkyl.

$C_{1-6}$Alkyl groups alone or as part of another group (for example $C_{1-6}$alkoxy), can be straight or branched, for example methyl, ethyl, n-propyl, i-propyl, i-butyl, s-butyl, n-butyl, n-pentyl, i-pentyl or n-hexyl. Preferably $C_{1-6}$alkyl groups are methyl or ethyl.

Preferably, $C_{1-6}$alkoxy groups are methoxy or ethoxy.

Halogen groups are fluorine, chlorine, bromine or iodine. Preferably halogen groups are chlorine or bromine.

Preferred compounds of the present invention include

(i) compounds of structure (I) in which $R^5$ and $R^6$ are both $C_{1-6}$alkyl, for example,
2-(5-chloro-4-dimethylamino-2-pyridylmethylsulphinyl)-5-methoxy-(1H)-benzimidazole
2-(3-chloro-4-dimethylamino-2-pyridylmethylsulphinyl)-5-methoxy-(1H)-benzimidazole
2-(3-methyl-5-chloro-4-dimethylamino-2-pyridylmethylsulphinyl)-5-methoxy-(1H)-benzimidazole
2-(3-chloro-5-methyl-4-dimethylamino-2-pyridylmethylsulphinyl)-5-methoxy-(1H)-benzimidazole
2-(5-bromo-4-dimethylamino-2-pyridylmethylsulphinyl)-5-methoxy-(1H)-benzimidazole

(ii) compounds of structure (I) in which $R^5$ and $R^6$ together form a morpholino or piperidino group, for example,
2-(3-chloro-4-morpholino-2-pyridylmethylsulphinyl)-5-methoxy-(1H)-benzimidazole
2-(3-chloro-5-methyl-4-morpholino-2-pyridylmethylsulphinyl)-5-methoxy-(1H)-benzimidazole
2-(5-chloro-4-piperidino-2-pyridylmethylsulphinyl)-5-methoxy-(1H)-benzimidazole
2-(3-chloro-r-piperidino-2-pyridylmethylsulphinyl)-5-methoxy-(1H)-benzimidazole
2-(3-methyl-5-chloro-4-piperidino-2-pyridylmethylsulphinyl)-5-methoxy-(1H)-benzimidazole
2-(3-chloro-5-methyl-4-piperidino-2-pyridylmethylsulphinyl)-5-methoxy-(1H)-benzimidazole
2-(3-bromo-4-piperidino-2-pyridylmethylsulphinyl)-5-methoxy-(1H)-benzimidazole
2-(3-methyl-5-bromo-4-piperidino-2-pyridylmethylsulphinyl)-5-methoxy-(1H)-benzimidazole
2-(5-bromo-4-piperidino-2-pyridylmethylsulphinyl)-5-methoxy-(1H)-benzimidazole.

(iii) compounds of structure (I) in which the substituent on the benzimidazole ring is a 1,1,2,2-tetrafluoroethoxy group or a difluoromethlylenedioxy group, for example
2-(3-chloro-5-methyl-4-piperidino-2-pyridylmethylsulphinyl)-5-(1,1,2,2-tetrafluoroethoxy)-(1H)-benzimidazole
2-(3-chloro-5-methyl-4-piperidino-2-pyridylmethylsulphinyl)-4,5-difluoromethylenedioxy-(1H)-benzimidazole
2-(3-chloro-4-piperidino-2-pyridylmethylsulphinyl)-5-(1,1,2,2-tetrafluoroethoxy)-(1H)-benzimidazole
2-(3-chloro-4-piperidino-2-pyridylmethylsulphinyl)-4,5-difluoromethylenedioxy-(1H)-benzimidazole
2-(3-chloro-5-methyl-4-dimethylamino-2-pyridylmethysulphinyl)-5-(1,1,2,2-tetrafluoroethoxy)-(1H)-benzimidazole, and
2-(3-chloro-5-methyl-4-dimethylamino-2-pyridylmethysulphinyl)-4,5-difluoromethylenedioxy-(1H)-benzimidazole
2-(3-methyl-5-bromo-4-piperidino-2-pyridylmethylsulphinyl)-5-(1,1,2,2-tetrafluoroethoxy-(1H)-benzimidazole
2-(3-methyl-5-bromo-4-piperidino-2-pyridylmethylsulphinyl)-4,5- difluoromethylenedioxy-(1H)-benzimidazole 2-(3-bromo-4-piperidino-2-pyridylmethylsulphinyl)-5-(1,1,2,2-tetrafluoroethoxy)-(1H)-benzimidazole
2-(5-bromo-4-piperidino-2-pyridylmethylsulphinyl)-5-(1,1,2,2- tetrafluoroethoxy)-(1H)-benzimidazole
2-(3-bromo-4-piperidino-2-pyridylmethylsulphinyl)-4,5- difluoromethylenedioxy-(1H)-benzimidazole
2-(5-bromo-4-piperidino-2-pyridylmethylsulphinyl)-4,5- difluoromethylenedioxy-(1H)-benzimidazole
and

(iv) the corresponding thioethers of the foregoing preferred compounds ie the analogous compounds of structure (I) in which n is 0.

Compounds of structure (I) in which n is 0, can form pharmaceutically acceptable acid addition salts with suitable organic and inorganic acids, the nature of which will be apparent to persons skilled in the art. For example, pharmaceutically acceptable salts can be formed by reaction with hydrochloric, sulphuric, sulphonic or phosphonic acids; aliphatic, alicyclic, aromatic or heterocyclic carboxyl or sulphonic acids; methionine, tryptophan, lysine or arginine and the like.

Compounds of structure (I) in which n is 1 can also form pharmaceutically acceptable acid addition salts, but in aqueous solution the salts are less stable than those formed with the compounds of structure (I) in which n is 0.

Compounds of structure (I) in which n is 1 can form basic salts by reaction with an appropriate base. Such salts include, for example, the sodium, potassium, lithium, calcium and magnesium salts which can be prepared by methods well known to those skilled in the art for example, the sodium, potassium and lithium salts can be prepared by reaction with sodium, potassium or lithium hydroxide in an aqueous or non-aqueous medium; and the calcium salts can be prepared by reaction with calcium chloride in an aqueous or non-aqueous medium.

Compounds of structure (I) in which n is 0 can also form basic salts but such salts are less stable than those prepared from compounds of structure (I) in which n is 1.

Compounds of structure (I) in which n is 1 have an asymmetric centre at the S atom and are thus optically active compounds. As such, these compounds exist as two optical isomers (enantiomers). In addition, compounds of structure (I) in which one or more of $R^2$, $R^3$ and $R^5$ to $R^8$ is a branched $C_{3-6}$alkyl group (either alone or as part of another group) may contain an additional asymmetic centre(s) due to the presence of the $C_{3-6}$alkyl group(s). Again, such compounds will exist as two (or more) optical isomers.

Both the pure enantiomers, racemic mixtures (50% of each enantiomer) and unequal mixtures of the two are included within the scope of the present invention. Further, all diasteriomeric forms possible (pure enantiomers and mixtures thereof) are within the scope of the invention.

It should be noted that for all the compounds of the present invention, the substituents $R^2$ and $R^3$ are considered to be equivalent at room temperature in solution. This is due to the tautomerism of the benzimidazole nucleus causing an equilibrium between the 2 possible forms.

In a further aspect the present invention provides a process for the preparation of a compound of structure (I) or a pharmaceutically acceptable salt thereof which comprises

a) reacting a compound of structure (II)

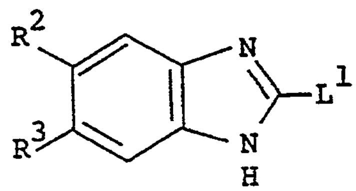

(II)

with a compound of structure (III)

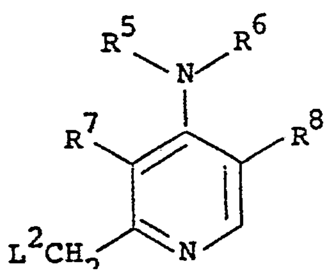

(III)

in which $R^2$, $R^3$ and $R^5$ to $R^8$ are as described for structure (I) and one of $L^1$ and $L^2$ is SH and the other a leaving group displaceable by a mercaptan;

(b) reacting a compound of structure (IV)

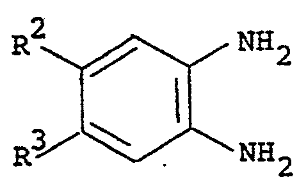

(IV)

in which $R^2$ and $R^3$ are as described for structure (I), with a compound of structure (V)

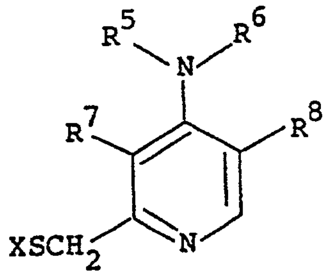

(V)

in which $R^5$ to $R^8$ are as described for structure (I), X is $CO_2H$ or $CSX^1$ and $X^1$ is halogen or $C_{1-4}$alkoxy;

(c) reacting a compound of structure (VI)

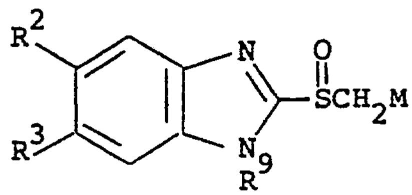

(VI)

in which $R^2$ and $R^3$ are as described for structure (I), $R^9$ is hydrogen or a protecting group and M is an alkali metal atom, with a compound of structure (VII)

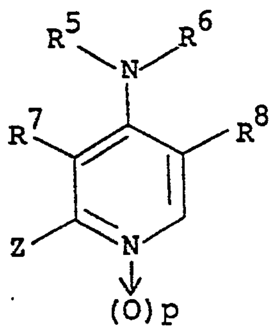

(VII)

in which $R^5$ to $R^8$ are as described for structure (I), Z is a leaving group and p is 0 or 1;

(d) reacting a compound of structure (VIII)

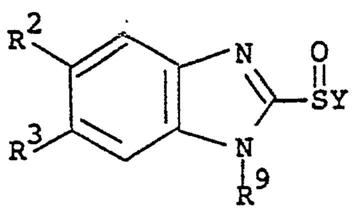

(VIII)

in which $R^2$ and $R^3$ are as described for structure (I), $R^9$ is hydrogen or a protecting group and Y is a leaving group, with a compound of structure (IX)

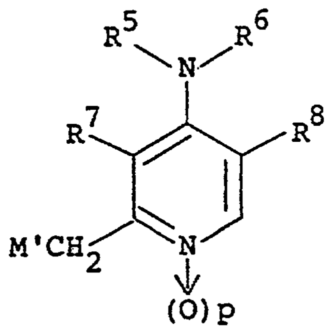

(IX)

in which $R^5$ to $R^8$ are as described or structure (I), p is 0 or 1 and M' is an alkali metal atom or the equivalent of an alkali metal atom,

and, optionally, where desired:

(i) oxidising a compound of structure (I) so formed in which n is 0 to a compound of structure (I) in which n is 1;
(ii) reducing a compound of structure (I) so formed in which n is 1 to a compound of structure (I) in which n is 0;
(iii) forming a pharmaceutically acceptable salt.

Suitable leaving groups L1 displaceable by mercaptan include halogen, for example chloro, bromo or iodo, aryl sulphonyloxy for example toluenesulphonyloxy, alkylsulphonyloxy for example methanesulphonyloxy, alkylmercapto, for example methylmercapto, or alkylsulphinyl, for example methylsulphinyl.

Suitable leaving groups $L^2$ are as described for $L^1$, and may also be $C_{1-4}$acyloxy, for example acetoxy, or hydroxy.

Suitable alkali metal atoms include, for example lithium, sodium or potassium.

Suitable leaving groups Z include, for example, halogen (preferably chloro) and hydroxy activated by esterification with, for example, an aryl or alkane sulphonic acid. Suitable sulphonic acids will be apparent to those skilled in the art, for example p-toluenesulphonic acid or methanesulphonic acid.

Suitable leaving groups Y are those groups which form a reactive sulphinic acid derivative together with the sulphinyl group to which it is attached, and include for example, $C_{1-4}$alkoxy, di-$C_{1-4}$alkylamino and $C_{1-4}$alkyl-mercapto.

Suitable groups M' which are equivalent to a metal atom include, for example, alkali earth metal atoms, (for example magnesium) which are substituted by a halogen atom (for example, bromine).

Suitable protecting groups $R^9$ are those conventional in the art for example as described in "Protective Groups in Organic Synthesis" T.W. Greene 1981 (Wiley). It will be appreciated that the group $R^9$ should not be cleavable under the conditions of reaction of compounds of structure (VIII) and (IX). Such groups include for example benzyl or trityl groups.

The reaction between compounds of structure (II) in which $L^1$ is SH and compounds of structure (III) in which $L^2$ is a leaving group can be carried out under basic conditions in the presence of an inert solvent at a temperature between ambient and the reflux temperature of the solvent.

Suitable solvents include lower alkanols, for example methanol or ethanol, mixtures of lower alkanols with water, or ethers for example dimethoxyethane or tehrahydrofuran.

Suitable bases will be apparent to those skilled in the art and include for example, alkali metal hydroxides, for example, sodium or potassium hydroxide, alkali metal alkoxides, for example potassium t-butoxide, alkali metal hydrides, for example sodium or potassium hydride, or organic tertiary amines, for example triethylamine.

Preferably the reaction is carried out at ambient temperature in ethanol as solvent, in the presence of sodium hydroxide solution.

It is to be noted, and will be apparent to persons skilled in the art that under basic conditions $L^2$ should be a group other than hydroxy or acetoxy, for example halogen, preferably chlorine.

Further, the reaction can be carried out under neutral conditions in the presence of an inert solvent at the reflux temperature of the solvent. Suitable solvents include those hereinbefore described.

Alternatively, when $L^2$ is hydroxy or $C_{1-4}$acyloxy, for example acetoxy, the reaction can be carried out under acidic conditions. Suitable acidic conditions will be well known to those skilled in the art, for example, under reflux in hydrobromic acid, optionally in the presence of acetic acid.

The reaction between compounds of structure (II) in which $L^1$ is a leaving group and compounds of structure (III) in which $L^2$ is SH can be carried out under basic conditions as described for the reaction between compounds of structure (II) in which $L^1$ is SH and compounds of structure (III) in which $L^2$ is a leaving group.

The reaction between compounds of structure (IV) and compounds of structure (V) can be carried out under acidic conditions in a suitable solvent at a temperature between ambient and reflux temperature of the solvent used.

Suitably the reaction is carried out in polar solvents, for example, lower alkanols, dimethyl sulphoxide, acetone, dimethylformamide or acetonitrile, optionally in the presence of water. Preferably the reaction is carried out in ethanol.

Suitably the reaction is carried out in the presence of a strong acid, for example hydrobromic or hydrochloric acid. Preferably the reaction is carried out in the presence of hydrochloric acid.

Preferably the reaction is carried out at the reflux temperature of the solvent.

The reaction between a compound of structure (VI) and a compound of structure (VII) can be carried out in an inert solvent at ambient or elevated temperature depending on the nature of groups M and Z. Suitable solvents include those solvents usually employed for the reaction of enolate ions with alkylating agents for example, benzene or toluene. Preferably, when M is lithium and Z is chlorine, the reaction is conducted in benzene at reflux temperature.

The reaction between a compound of structure (VIII) and a compound of structure (IX) can be carried out under conditions normally used for organometallic reactions as will be well known to those skilled in the art.

The products of reactions (a) to (c) are compounds of structure (I) in which n is 0. These products can be oxidised to compounds of structure (I) in which n is 1 by reaction with an oxidising agent. Suitable oxidising agents include, for example, nitric acid, hydrogen peroxide, peracids, peresters, ozone, dinitrogen tetroxide, iodosobenzene, N-halosuccinamide, 1-chlorobenzotriazole, hypohalites, for example sodium hypochlorite or t-butyl hypochlorite, diazabicylo [2,2,2]- octane bromine complex, sodium metaperiodate, selenium dioxide, manganese dioxide, chromic acid, ceric ammonium nitrate, bromine, chlorine, or sulphuryl chloride. Preferably the oxidising agent is m-chloroperbenzoic acid.

The oxidation reaction is carried out under conditions known in the art for the oxidation of thiols to sulphoxides. Suitably, the reaction is carried out in an inert solvent at a temperature of between -70° and the boiling point of the solvent used. Suitable solvents include aromatic or chlorinated hydrocarbons, for example benzene, toluene dichloromethane or chloroform, esters, for example ethyl acetate, or ethers, for example dioxan. Preferably, the reaction is carried out in dichloromethane at a temperature of between -50° and +20°C.

The compounds of structure (I) are obtained either as the free base, or in the form of a salt depending on the choice of starting materials and reaction conditions. If the free compound is obtained it can be converted into a salt by standard techniques well-known to those skilled in the art, for example by dissolving the compound in a suitable solvent and adding the desired acid or base; alternatively, if a salt is obtained it can be converted into the free compound, again by standard techniques, for example by treatment with an appropriate acid or base.

Racemic mixtures may be produced and can be separated by standard techniques e.g. recrystallisation from optically active solvent or by high performance liquid affinity chromatography as described by S. Allenmark, B. Bomgren, H. Baren and P-O Lagerstrom in Analytical Biochemistry, 136, 293-7, 1984.

The intermediate of structure (IV) and the intermediate benzimidazole structures (II), (VI) and (VIII) are known and can be prepared by methods analogous to those known in the art. For example, compounds of structure (II) in which $L^1$ is SH can be prepared by reacting the corresponding compounds of structure (IV) with carbon disulphide in the presence if alkali metal hydroxides, or with potassium ethylxanthate (Org. Syn., 30, 56) or thiophosgene. Compounds of structure (II) in which $L^1$ is a leaving group, for example halogen can be obtained from the corresponding compounds of structure (II) in which $L^1$ is hydroxy by treatment with for example, phosphorous oxychloride. The compounds of structure (II) in which $L^1$ is hydroxy can be prepared by reacting compounds of structure (IV) with phosgene. Compounds of structure (IV) can be prepared by methods analogous to those described in EP 127763A, DE 2848531, CA, 60, 13352h, 1964 and Liebigs Ann.Chem., 730, 16-30, 1969.

Compounds of structure (VI) can be prepared by methylation, oxidation and subsequent deprotection of compounds of structure (II) in which $L^1$ is SH using, for example, alkali metal hydroxides or alcoholates.

The intermediates of structures (III), (V), (VII) and (IX) are novel and provide a further aspect of the invention. They can be prepared by methods analogous to those well known in the art as described in "The Chemistry of Heterocyclic Compounds - Pyridine and its Derivatives", Pts. 2 and 3, E. Klingsberg Ed., Interscience Publishers, 1962. For example,

(i) compounds of structure (III) in which $L^2$ is hydroxy and $R^5$ and $R^6$ are not both hydrogen can be prepared by the reactions outlined in Scheme 1.

Scheme 1

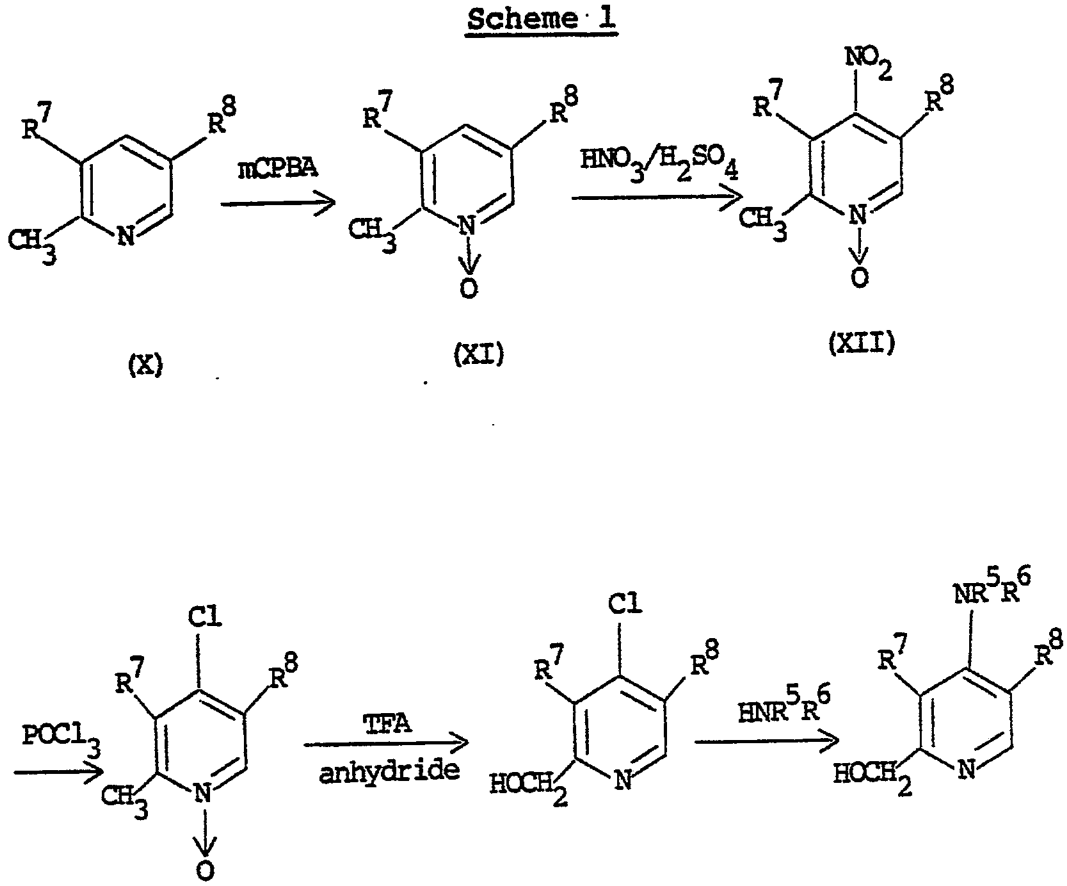


(X) (XI) (XII)

(XIII) (XIV)

In Scheme 1, the 3- or 5-halo compounds of structure (X) are known or can be prepared by standard methods.

(ii) compounds of structure (III) in which L2 is hydroxy, $R^5$ and $R^6$ are not both hydrogen, one of $R^7$ and $R^8$ is bromine and the other is hydrogen or $C_{1-6}$alkyl can also be prepared by the methods outlined in Scheme 2.

Scheme 2

$R^7 = R^8 =$ hydrogen

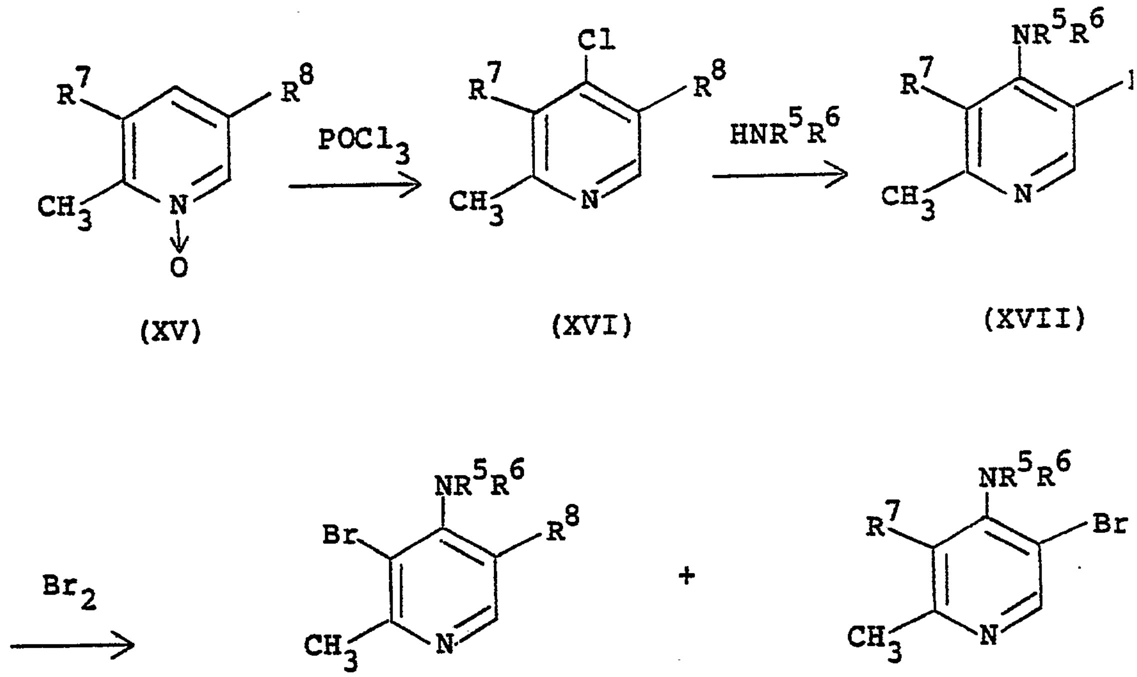


(XV) (XVI) (XVII)

(XVIII) 3 : 1 (XIX)

Separation of the 3- and 5-bromo intermediates (XVIII) and (XIX) followed by oxidation to the corresponding N-oxides and rearrangement using, for example, acetic anhydride gives the required compounds of structure (III) in which one of $R^7$ and $R^8$ is bromine and the other is hydrogen. If, in the starting material of structure (XV) one of $R^7$ and $R^8$ is $C_{1-6}$alkyl and the other is hydrogen, then the bromination reaction gives essentially a single product, namely the 3- bromo-5-alkyl compounds (XVIII, $R^8$ = $C_{1-6}$alkyl) or 3-alkyl-5-bromo compounds (XIX, $R^7$ = $C_{1-6}$alkyl). These products can be oxidised and rearranged as hereinbefore described to the desired compounds of structure (III) in which one of $R^7$ and $R^8$ is bromine and the other is $C_{1-6}$alkyl.

(iii) compounds of structure (III) in which $L^2$ is hydroxy, $R^5$ and $R^6$ are not both hydrogen, $R^7$ is chlorine and $R^8$ is hydrogen or $C_{1-6}$alkyl can be prepared by the reactions outlined in Scheme 3.

## Scheme 3

$R^7$, = hydrogen, $R^8$ = hydrogen or $C_{1-6}$alkyl

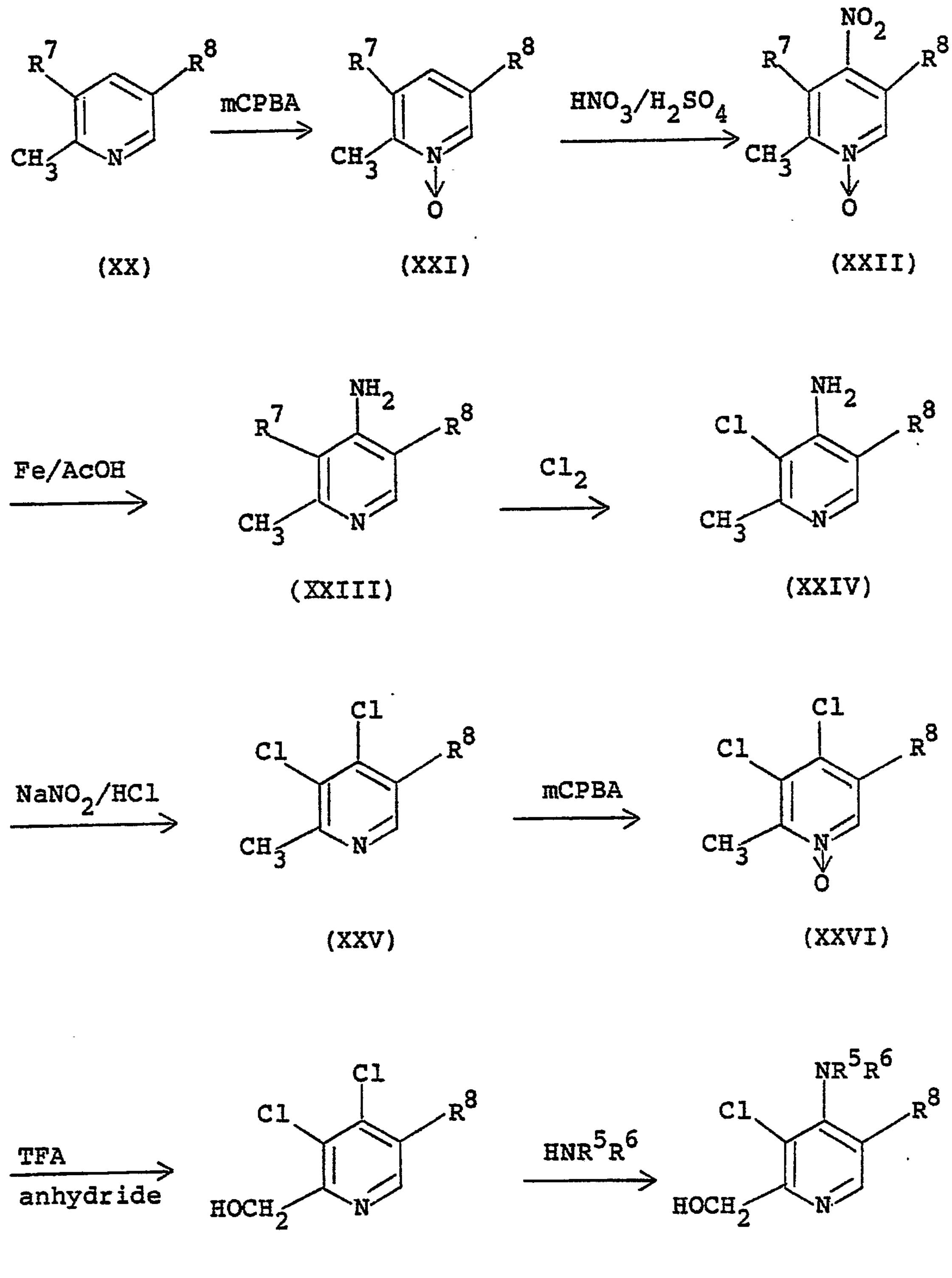


(XX) (XXI) (XXII)

(XXIII) (XXIV)

(XXV) (XXVI)

(XXVII) (XXVIII)

(iv) compounds of structure (III) in which $L^2$ is hydroxy and $R^5$ and $R^6$ are both hydrogen can be prepared by reduction of compounds of structure (XII) using for example Raney Nickel/hydrazine, followed by rearrangement of the 4-amino-N-oxide intermediate so formed, with, for example acetic anhydride.

Treatment of the compounds of structure (III) in which $L^2$ is OH with halogenating agents such as thionyl chloride or O-acylating agents such as p-toluenesulphonyl chloride gives the required compounds of structure (III) in which $L^2$ is halogen or sulphonyloxy respectively.

Compounds of structure (III) in which $L^2$ is SH can be prepared from compounds of structure (III) in which $L^2$ is a leaving group for example halogen, by reaction with, for example, NaSH.

Compounds of structure (V) can be prepared from the corresponding compounds of structure (III); for example, compounds of structure (V) in which X is CSCl can be prepared by reaction of a compound of structure (III) in which $L^2$ is SH with thiophosgene.

Compounds of structure (VII) can be prepared by methods analogous to those described in Roszniki Chem., 35, 475, 1961.

In a further aspect the present invention provides compounds of structure (I) and pharmaceutically acceptable salts thereof for use in therapy. The compounds of structure (I) and their pharmaceutically acceptable salts inhibit exogenously and endogenously stimulated gastric acid secretion and are useful in the treatment of gastro-intestinal diseases in mammals, in particular humans. Such diseases include, for example, gastric and duodenal ulcers, and Zollinger-Ellison Syndrome.

Further, the compounds of structure (I) can be used in the treatment of other disorders where a cytoprotective and/or anti- secretory effect is desirable for example in patients with gastritis, NSAID induced gastritis, gastric ulcers, acute upper intestinal bleeding, and in patients with a history of chronic and excessive alcohol consumption.

It is believed that, after administration to mammals, compounds of structure (I) in which n is 0 exert their anti-secretory and cytoprotective activities after conversion into compounds of structure (I) in which n is 1.

Furthermore it is believed that compounds of structure (I) in which n is 1, after administration to mammals, exert their anti-secretory activity after transformation under acid conditions into another chemically reactive species. Active species so generated from compounds of structure (I) are within the scope of the present invention.

In therapeutic use, the compounds of the present invention are usually administered in a standard pharmaceutical composition. The present invention therefore provides in a further aspect pharmaceutical compositions comprising a compound of structure (I) or a pharmaceutically acceptable salt thereof and a pharmaceutically acceptable carrier.

The compounds of structure (I) and their pharmaceutically acceptable salts which are active when given orally can be formulated as liquids, for example syrups, suspensions or emulsions, tablets, capsules and lozenges.

A liquid formulation will generally consist of a suspension or solution of the compound or pharmaceutically acceptable salt in a suitable liquid carrier(s) for example, ethanol, glycerine, non-aqueous solvent, for example polyethylene glycol, oils, or water with a suspending agent, preservative, flavouring or colouring agent.

A composition in the form of a tablet can be prepared using any suitable pharmaceutical carrier(s) routinely used for preparing solid formulations. Examples of such carriers include magnesium stearate, starch, lactose, sucrose and cellulose.

A composition in the form of a capsule can be prepared using routine encapsulation procedures. For example, pellets containing the active ingredient can be prepared using standard carriers and then filled into a hard gelatin capsule; alternatively, a dispersion or suspension can be prepared using any suitable pharmaceutical carrier(s), for example aqueous gums, celluloses, silicates or oils and the dispersion or suspension then filled into a soft gelatin capsule.

Compounds of structure (I) in which n is 1 are susceptible to decomposition in acid media, and thus tablets and capsules containing such compounds are preferably provided with an enteric coating to protect the compound from acid degradation in the stomach or capsules used which are inherently acid resistant. Alternatively, the enteric coating can be provided by coating pellets containing the active ingredient before filling them into the hard gelatin capsule. Suitable enteric coating materials are those well known in the art of pharmacy and include for example shellac or anionic-film forming polymers such as cellulose acetate phthalate and hydroxypropylmethyl cellulose phthalate and the like, optionally in the presence of a plasticizer.

Typical parenteral compositions consist of a solution or suspension of the compound or pharmaceutically acceptable salt in a sterile aqueous carrier or parenterally acceptable oil, for example polyethylene glycol, polyvinyl pyrrolidone, lecithin, arachis oil or sesame oil. Alternatively, the solution can be lyophilised and then reconstituted with a suitable solvent just prior to administration.

A typical suppository formulation comprises a compound of formula (I) or a pharmaceutically acceptable salt thereof which is active when administered in this way, with a binding and/or lubricating agent such as polymeric glycols, gelatins or cocoa butter or other low melting vegetable or synthetic waxes or fats.

Preferably the composition is in unit dose form such as a tablet or capsule.

Each dosage unit for oral administration contains preferably from 1 to 250 mg (and for parenteral administration contains preferably from 0.1 to 25 mg) of a compound of the formula (I) or a pharmaceutically acceptable salt thereof calculated as the free acid.

The present invention also provides a method of inhibiting gastric acid secretion which comprises administering to a mammal in need thereof an effective amount of a compound of the formula (I) or a pharmaceutically acceptable salt thereof.

The pharmaceutically acceptable compounds of the invention will normally be administered to a subject for the treatment of gastro intestinal diseases and other conditions caused or exacerbated by gastric acidity. The daily dosage regimen for an adult patient may be, for example, an oral dose of between 1 mg and 500 mg, preferably between 1 mg and 250 mg, or an intravenous, subcutaneous, or intramuscular dose of between 0.1 mg and 100 mg, preferably between 0.1 mg and 25 mg, of the compound of the formula (I) or a pharmaceutically acceptable salt thereof calculated as the free base, the compound being administered 1 to 4 times per day. Suitably the compounds will be administered for a period of continuous therapy, for example for a week or more.

In addition, the compounds of the present invention can be co- administered with further active ingredients, such as antacids (for example magnesium carbonate or hydroxide and aluminium hydroxide), non steroidal anti- flammatory drugs (for example indomethacin, aspirin or naproxen), steroids, or nitrite scavengers (for example ascorbic acid or aminosulphonic acid), or other drugs used for treating gastric ulcers (for example pirenzipine, prostanoids for example 16,16 dimethyl $PGE_2$, or histamine $H_2$-antagonists (for example, cimetidine).

The following examples illustrate the invention. Temperatures are recorded in degrees centigrade.

Example 1A

Preparation of 4-amino-3-chloro-2-picoline

Precondensed chlorine (ca. 20 ml) was evaporated into a stirred, ice cooled solution of 4-amino-2-picoline (44g) in sulphuric acid (1l, 50% v/v) in a flask fitted with a dri-cold trap. After stirring for a further 2 hours the solution was basified (NaOH) and extracted with ether. The extract was dried ($K_2CO_3$), treated with charcoal and reduced to a small volume. Pet. ether (40/60) was added to give a solid which was recrystallised from chloroform/pet. ether (60/80) to give 4-amino-3-chloro-2-picoline, 37.2g, m.p. 116-18°.

Example 1B

Preparation of 3,4-dichloro-2-picoline

Sodium nitrite (36g) was added in portions to a stirred solution of 4-amino-3-chloro-2-picoline (24.85g) in conc. hydrochloric acid (750 ml) cooled to 0-5°. After 1 hour at 0-5° and a further 2 hours at room temperature the mixture was basified (NaOH), allowing the temperature to rise to ca. 50°, and extracted with ether. After drying ($K_2CO_3$), the extract was evaporated to dryness to give 3,4-dichloro-2-picoline (26.5g) as a low melting solid.

Example 1C

Preparation of 3,4-dichloro-2-hydroxymethylpyridine

m-Chloroperbenzoic acid (32.63g) in dichloromethane (400 ml) was added dropwise to a solution of 3,4-dichloro-2-picoline (25.53 g)in dichloromethane (100ml) maintaining the temperature at 20-25°. After standing for 16 hours at room temperature the solution was washed with 1N NaOH, dried ($K_2CO_3$) and filtered to give a pale yellow solution. Trifluoroacetic anhydride (30 ml) was added dropwise to this solution maintaining the temperature at 15-20°. After standing for 2 days at room temperature, methanol (100 ml) was added and the solution evaporated under reduced presure. The residue was treated with aqueous sodium carbonate and extracted into dichloromethane. After drying ($K_2CO_3$) and removal of solvent, the residue was recrystallised from pet. ether (60/80) to give 3,4-dichloro-2-hydroxymethylpyridine, 15.76 g, m.p. 66-8°.

Example 1D

Preparation of 4-morpholino-3-chloro-2-hydroxymethylpyridine

Morpholine (7.34 ml) and 3,4-dichloro-2-hydroxymethylpyridine (3.0 g) were heated together in a bomb at 180° for 4 hours. After cooling, the mixture was taken up in ethanol and evaporated under reduced pressure to remove excess amine. The residue was taken up in water and extracted with chloroform. After drying ($K_2CO_3$) and removal of solvent, the residue was chromatographed (silica gel, CH-

$Cl_3$) to give an oil which was crystallised from petroleum ether (60/80) to give 4-morpholino-3-chloro-2-hydroxymethylpyridine, 3.24 g, m.p. 80-2°.

Example 1E

Preparation of 4-morpholino-3-chloro-2-chloromethylpyridine hydrochloride

Thionyl chloride (3 ml) in chloroform (25 ml) was added dropwise to a solution of 4-morpholino-3-chloro-2-hydroxymethylpyridine (3.09 g) in chloroform (25 ml) cooled in an ice/salt bath. Following the addition the ice bath was removed and the mixture stirred for a further 1.5 hours. The volume of the solution was reduced and ether added to give 4- morpholino-3-chloro-2-chloromethylpyridine hydrochloride, 3.77 g, m.p. 200-2°.

Example 1F

Preparation of 2-(4-morpholino-3-chloro-2-pyridylmethylthio)-5- methoxy-(1H)-benzimidazole

5N Sodium hydroxide (5.68 ml) was added dropwise to a stirred solution of 4-morpholino-3-chloro-2-chloromethyl- pyridine hydrochloride (3.66 g) and 5-methoxy-2-mercapto- benzimidazole (2.33 g). After standing overnight the solution was evaporated under reduced pressure and the residue triturated with water. The solid thus obtained was recrystallised from ethanol to give 2-(4-morpholino-3-chloro-2- pyridyl-methylthio)-5-methoxy-(1H)-benzimidazole, 4.38 g, m.p. 124-25°.

Example 2

Preparation of 2-(4-morpholino-3-chloro-2-pyridylmethyl- sulphinyl)-5-methoxy-(1H)-benzimidazole

A solution of m-chloroperbenzoic acid (1.61 g) in dichloromethane (75 ml) was added dropwise to a stirred solution of 2-(4- morpholino-3-chloro-2-pyridylmethylthio)-5-methoxy-(1H)-benzimidazole (3.35 g) in dichloromethane (150 ml) cooled to -35°. After 1 hour, ammonia was passed through the solution for 5 minutes and the precipitate filtered off. The solution was evaporated under reduced pressure and the residue chromatographed (silica gel, 2% $CHCl_3/MeOH-NH_3$) to give an oil which was crystallised from ethanol to give 2-(4-morpholino-3-chloro-2-pyridylmethylsulphinyl)-5-methoxy-(1H) -benzimidazole, (2.6 g).

$C_{18}H_{19}ClN_4O_3S$
Found C 53.39, H 4.79, N 13.62, S 7.75, Cl 8.84
Requires C 53.13, H 4.71, N 13.77, S 7.88, Cl 8.71

Example 3A

Preparation of 4-amino-3-chloro-2,5-dimethylpyridine

Substituting 4-amino-2,5-dimethylpyridine (35 g) for 4-amino-2 -picoline and using corresponding molar proportions of the other reagents in the method of Example 1A gave 4-amino-3-chloro-2,5-dimethylpyridine, 26 g, m.p. 82-3°.

Example 3B

Preparation of 3,4-dichloro-2,5-dimethylpyridine

Substituting 4-amino-3-chloro-2,5-dimethylpyridine (26 g) for 4 -amino-3-chloro-2-picoline and using corresponding molar proportions of the other reagents in the method of Example 1B gave 3,4-dichloro-2,5-dimethylpyridine, 26.8 g, as an oil.

Example 3C

Preparation of 3,4-dichloro-5-methyl-2-hydroxymethylpyridine

Substituting 3,4-dichloro-2,5-dimethylpyridine (26.8 g) for 3,4-dichloro-2-picoline and using corresponding molar proportions of the other reagents in the method of Example 1C gave 3,4-dichloro-5-methyl-2-hydroxymethylpyridine, 22.5g, m.p. 77-8° from ether/pet. ether (40/60).

Example 3D

Preparation of 4-morpholino-3-chloro-5-methyl-2- hydroxymethylpyridine

Substituting 3,4-dichloro-5-methyl-2- hydroxymethylpyridine (4 g) for 3,4-dichloro-2-hydroxymethylpyridine and using corresponding molar proportions of the other reagents, in the method of Example 1D gave 4-morpholino- 3-chloro-5-methyl-2-hydroxymethylpyridine, 4.49 g, m.p. 54-5° from pet. ether (60/80).

Example 3E

Preparation of 4-morpholino-3-chloro-5-methyl-2- chloromethylpyridine Hydrochloride

Substituting 4-morpholino-3-chloro-5-methyl-2- hydroxymethylpyridine (4.0 g) for 4-morpholino-3-chloro-2- hydroxymethylpyridine and using corresponding molar proportions of the other reagents in the method of Example 1E gave 4-morpholino-3-chloro-5-methyl-2- chloromethylpyridine hydrochloride, 4.9 g, m.p. 176-9°.

Example 3F

Preparation of 2-(4-morpholino-3-chloro-5-methyl-2- pyridylmethylthio)-5-methoxy-(1H)-benzimidazole

Substituting 4-morpholino-3-chloro-5-methyl-2- chloromethylpyridine hydrochloride (4.0g) for 4-morpholino-3- chloro-2-chloromethylpyridine hydrochloride and using corresponding molar proportions of the other reagents in the method of Example 1F gave 2-(4-morpholino-3-chloro-5- methyl-2-pyridylmethylthio)-5-methoxy-(1H)-benzimidazole, 3.96g, m.p. 162-4° from acetonitrile.

Example 4

Preparation of 2-(4-morpholino-3-chloro-5-methyl-2- pyridylmethylsulphinyl)-5-methoxy-(1H)-benzimidazole

Substituting 2-(4-morpholino-3-chloro-5-methyl-2- pyridylmethylthio)-5-methoxy-(1H)-benzimidazole (3.8g) for 2-(4- morpholino-3-chloro-2-pyridylmethylthio)-5-methoxy-(1H)- benzimidazole and using corresponding molar proportions of the other reagents in the method of Example 2 gave, after chromatography (alumina $CHCl_3$/MeOH 0-1%), 2-(4-morpholino- 3-chloro-5-methyl-2-pyridylmethylsulphinyl)-5-methoxy-(1H)- benzimidazole, 1.09 g, m.p. 147-48° from acetonitrile.
$C_{19}H_{21}ClN_4O_3S$
Found C 54.25, H 5.08, N 13.25, S 7.6, Cl 8.68
Requires C 54.22, H 5.03, N 13.31, S 7.62, Cl 8.42

Example 5A

Preparation of 4-piperidino-3-chloro-5-methyl-2- hydroxymethylpyridine

Substituting piperidine for morpholine in the method of Example 3D, gave 4-piperidino-3-chloro-5-methyl-2- hydroxymethylpyridine (80%) as an oil.

Example 5B

Preparation of 4-piperidino-3-chloro-5-methyl-2- chloromethylpyridine hydrochloride

Substituting 4-piperidino-3-chloro-5-methyl-2- hydroxymethylpyridine (2.9g) for 4-morpholino-3-chloro-5-methyl- 2-hydroxymethylpyridine and using corresponding molar proportions of the other reagents in the method of Example 3E gave 4-piperidino-3-chloro-5-methyl-2- chloromethylpyridine hydrochloride, 3.5g, m.p. 178-80°

Example 5C

Preparation of 2-(4-piperidino-3-chloro-5-methyl-2- pyridylmethylthio)-5-methoxy-(1H)-benzimidazole

Substituting 4-piperidino-3-chloro-5-methyl-2- chloromethylpyridine hydrochloride (3 g) for 4-morpholino-3- chloro-2-chloromethylpyridine hydrochloride and using corresponding molar proportions of the other reagents in the method of Example 3F gave 2-(4-piperidino-3- chloro-5-methyl-2-pyridylmethylthio)-5-methoxy-(1H)- benzimidazole, 3.1 g, m.p. 154-56° from acetonitrile.

Example 6

Preparation of 2-(4-piperidino-3-chloro-5-methyl-2- pyridylmethylsulpinyl)-5-methoxy-(1H)-benzimidazole

Substituting 2-(4-piperidino-3-chloro-5-methyl-2-pyridylmethylthio) -5-methoxy-(1H)-benzimidazole (2.3 g) for 2-(4-morpholino-3 -chloro-5-methyl-2-pyridylmethylthio)-5- methoxy-(1H)-benzimidazole and using corresponding molar proportions of the other reagents, in the method of Example 4, gave 2-(4-piperidino-3-chloro-5-methyl-2- pyridylmethylsulphinyl)-5-methoxy-(1H)-benzimidazole, 0.78 g, m.p. 151-52° from acetonitrile.
$C_{20}H_{23}ClN_4O_2S$
Found C 57.17, H 5.47, N 13.33, S 7.6, Cl 8.54
Requires C 57.34, H 5.53, N 13.37, S 7.65, Cl 8.46

Example 7A

Preparation of 4-piperidino-3-chloro-2-hydroxymethylpyridine

Substituting piperidine (7.17 g) for morpholine and using corresponding molar proportions of the other reagents, in the method of Example 1D, gave 4-piperidino-3-chloro-2- hydroxymethyl pyridine, 2.7 g, m.p. 68-70° from pet. ether (40/60).

Example 7B

Preparation of 4-piperidino-3-chloro-2-chloromethylpyridine hydrochloride

Substituting 4-piperidino-3-chloro-2-hydroxymethyl pyridine (3.43 g) for 4-morpholino-3-chloro-2-hydroxymethyl pyridine and using corresponding molar proportions of the other reagents in the method of Example 1E, gave 4-piperidino-3 -chloro-2-chloromethyl pyridine hydrochloride, 4.02 g, m.p. 192-4°.

Example 7C

Preparation of 2-(4-piperidino-3-chloro-2-pyridylmethylthio) -5-methoxy-(1H)-benzimidazole

Substituting 4-piperidino-3-chloro-2-chloromethylpyridine hydrochloride (3.87 g) for 4-morpholino-3-chloro-2-chloromethyl pyridine hydrochloride and using corresponding molar proportions of the other reagents in the method of Example 1F, gave 2-(4-piperidino-3 -chloro-2-pyridylmethylthio)-5-methoxy-(1H)-benzimidazole, 4.53 g, m.p. 145-7°.

Example 8

Preparation of 2-(4-piperidino-3-chloro-2-pyridylmethylsulphinyl) -5-methoxy-(1H)-benzimidazole

Substituting 2-(4-piperidino-3-chloro-2-pyridylmethylthio)- 5-methoxy-(1H)-benzimidazole (4.12 g) for 2-(4-morpholino- 3-chloro-2-pyridylmethylthio)-5-methoxy-(1H)-benzimidazole and using corresponding molar proportions of the other reagents, in the method of Example 2, gave 2-(4-piperidino- 3-chloro-2-pyridylmethylsulphinyl)-5-methoxy-(1H)- benzimidazole, 1.51 g.
$C_{19}H_{21}ClN_4O_2S$
Found C 56.72, H 5.28, N 13.80, S 7.79, Cl 8.67
Requires C 56.36, H 5.23, N 13.84, S 7.92, Cl 8.76

Example 9A

Preparation of 4-dimethylamino-3-chloro-5-methyl-2- hydroxymethylpyridine

3,4-dichloro-5-methyl-2-hydroxymethylpyridine (4 g) was dissolved in 33% dimethylamine in ethanol (17 ml), placed in a sealed vessel and heated to 200°C for 4 hours. On cooling, the reaction mixture was evaporated under reduced presure and the residue dissolved in water (15 ml) and ether extracted. Etherial extracts were dried ($K_2CO_3$), filtered and stripped to yield 4-dimethylamino-3-chloro-5- methyl-2-hydroxymethylpyridine, 4.08 g as an oil.

Example 9B

Preparation of 4-dimethylamino-3-chloro-5-methyl-2-chloromethylpyridine hydrochloride

Substituting 4-dimethylamino-3-chloro-5-methyl-2-hydroxymethylpyridine (3.5 g) for 4-morpholino-3-chloro-5-methyl- 2-hydroxymethylpyridine and using corresponding molar proportions of the other reagents in the method of Example 5E gave 4-dimethylamino-3-chloro-5-methyl-2-chloro- methylpyridine hydrochloride, 4.0 g, m.p. 162-163°.

Example 9C

Preparation of 2-(4-dimethylamino-3-chloro-5-methyl-2- pyridylmethylthio)-5-methoxy-(1H)-benzimidazole

Substituting 4-dimethylamino-3-chloro-5-methyl-2-chloromethylpyridine hydrochloride (3.9 g) for 4-morpholino-3-chloro-5-methyl-2 -pyridylmethylpyridine hydrochloride and using corresponding molar proportions of the other reagents in the method of Example 5F gave 2-(4-dimethyl- amino-3-chloro-5 -methyl-2-pyridylmethylthio)-5-methoxy-(1H)-benzimidazole, 4.2 g, m.p. 109-110° from acetonitrile.

Example 10

Preparation of 2-(4-dimethylamino-3-chloro-5-methyl-2- pyridylmethylsulphinyl)-5-methoxy-(1H)-benzimidazole

Substituting 2-(4-dimethylamino-3-chloro-5-methyl-2- pyridylmethylthio)-5-methoxy-(1H)-benzimidazole (1.6 g) for 2-(4 -morpholino-3-chloro-5-methyl-2-pyridylmethyl-thio)-5-methoxy-(1H) -benzimidazole and using corresponding molar proportions of the other reagents, in the method of Example 4, gave 2-(4-dimethylamino-3-chloro-5-methyl-2- pyridylmethylsulphinyl)-5-methoxy-(1H)-benzimidazole, 0.94 g, m.p. 147-149° (from acetonitrile).
$C_{17}H_{19}ClN_4O_2S$
Found C 53.69, H 5.02, N 14.70, Cl 9.37, S 8.43
Requires C 53.89, H 5.05, N 14.79, Cl 9.36, S 8.46

Example 11A

Preparation of 4-Piperidino-2-picoline

Piperidine (31.7 ml) and 4-chloro-2-picoline (13.65 g) were heated together in a bomb at 170° for 4.5 hours. After cooling, the mixture was taken up in water and extracted with ether. After drying ($K_2CO_3$) and stripping, the residue was distilled to give 4-piperidino-2-picoline, 15.45 g. b.p. 105-10° (0.1mm).

Example 11B

Preparation of 4-piperidino-5-bromo-2-picoline and 4-piperidino-3-bromo-2-picoline

A solution of bromine (9.3 ml) in dimethylformamide (50 ml) was added dropwise to a stirred mixture of 4-piperidino-2-picoline (15 g), potassium carbonate (23.5 g) in dimethylformamide (50 ml) at 25-30°. After 3.5 hours, the mixture was stripped and the residue taken up in water, adjusted to pH13 with 40% aqueous NaOH, and extracted with ether. After drying ($K_2CO_3$) and stripping, the residue was chromatographed (silica gel, n-hexane:ether) to give 4-piperidino-5-bromo-2-picoline, 11.18 g, as an oil and 4-piperidino-3-bromo-2-picoline, 3.5 g, as an oil.

Example 11C

Preparation of 4-piperidino-5-bromo-2-picoline-N-oxide

m-Chloroperbenzoic acid (7.3 g) in dichloromethane (100 ml) was added dropwise to a stirred solution of 4-piperidino-5- bromo-2-picoline. After 16 hours, the solution was washed with 10% aqueous sodium carbonate, dried ($K_2CO_3$) and stripped. The residue was chromatographed (silica gel, chloroform:methanol) to give 4-piperidino-5-bromo-2- picoline-N-oxide, 6.79 g, m.p. 115-6° from ether.

Example 11D

Preparation of 4-piperidino-5-bromo-2-hydroxymethylpyridine

4-piperidino-5-bromo-2-picoline-N-oxide (4.66 g) and acetic anhydride (25 ml) were heated at 100° for 1.25 hours. The solution was stripped, treated with toluene (30 ml) and stripped again. 2N Hydrochloric acid (35 ml) was added and the mixture heated at 100° for 2 hours. After cooling, 40% aqueous NaOH was added to pH13 and the solution extracted with ether. After drying ($K_2CO_3$) and stripping, the residue was chromatographed (silica gel, chloroform:methanol) to give 4 piperidino-5-bromo-2- hydroxymethylpyridine, 2.38 g, m.p. 118-9°, from ether.

Example 11E

Preparation of 4-piperidino-5-bromo-2-chloromethylpyridine hydrochloride

Substituting 4-piperidino-5-bromo-2-hydroxymethylpyridine (3.0 g) for 4-morpholino-3-chloro-2-hydroxymethylpyridine and using corresponding molar proportions of the other reagents in the method of Example 3E gave 4-piperidino-5- bromo-2-chloromethyl pyridine hydrochloride, 3.47 g, m.p. 155-7° by ether trituration.

Example 11F

Preparation of 2-(4-piperidino-5-bromo-2-pyridylmethylthio) -5-methoxy-(1H)-benzimidazole

Substituting 4-piperidino-5-bromo-2-chloromethyl pyridine hydrochloride (3.44g) for 4-morpholino-3-chloro-2- chloromethylpyridine hydrochloride and using corresponding molar proportions of the other reagents in the method of Example 3F gave after chromatography (silica gel, chloroform:methanol) 2-(4-piperidino-5-bromo-2 -pyridylmethylthio) -5-methoxy-(1H)-benzimidazole, 4.39 g, as an oil.

Example 12

Preparation of 2-(4-piperidino-5-bromo-2-pyridylmethylsulphinyl) -5-methoxy-(1H)-benzimidazole

Substituting 2-(4-piperidino-5-bromo-2-pyridylmethylthio)- 5-methoxy-(1H)-benzimidazole (3.83 g) for 2-(4-morpholino- 3-chloro-2-pyridylmethylthio-5-methoxy-(1H)-benzimidazole and using corresponding molar proportions of the other reagents in the method of Example 2 gave after chromatography (silica gel, chloroform:methanol-ammonia) 2-(4-piperidino-5-bromo-2 -pyridylmethylsulphinyl)-5-methoxy-(1H)-benzimidazole, 3.15 g, m.p. 114-8°C, from ether.
$C_{19}H_{21}BrN_4O_2S$
Found C 50.48, H 4.78, N 12.30, S 7.13, Br 17.44
Requires C 50.78, H 4.71, N 12.47, S 7.14, Br 17.78

Example 13A

Preparation of 4-piperidino-3-bromo-2-picoline-N-oxide

Substituting 4-piperidino-3-bromo-2-picoline (3.46 g) for 4-piperidino-5-bromo-2-picoline and using corresponding molar proportions of the other reagents in the method of Example 11C gave 4-piperidino-3-bromo-2-picoline-N-oxide, 2.5 g, m.p. 98-100° from pet. ether (60/80).

Example 13B

Preparation of 4-piperidino-3-bromo-2-hydroxymethylpyridine

Substituting 4-piperidino-3-bromo-2-picoline-N-oxide (2.42 g) for 4-piperidino-5-bromo-2-picoline-N-oxide and using corresponding molar proportions of the other reagents in the method of Example 11D gave 4-piperidino-3-bromo-2-hydroxymethylpyridine, 1.19 g, m.p. 51-3° from pet. ether (40/60).

Example 13C

Preparation of 4-piperidino-3-bromo-2-chloromethylpyridine hydrochloride

Substituting 4-piperidino-3-bromo-2-hydroxymethylpyridine (0.96 g) for 4-morpholino-3-chloro-2-hydroxymethylpyridine and using corresponding molar proportions of the other reagents in the method of Example 1E gave 4-piperidino-3- bromo-2 -chloromethylpyridine hydrochloride, 1.08 g, m.p. 182-4°C by ether trituration.

Example 13D

Preparation of 2-(4-piperidino-3-bromo-2-pyridylmethylthio) -5-methoxy-(1H)-benzimidazole

Substituting 4-piperidino-3-bromo-2-chloromethylpyridine hydrochloride (1.07g) for 4-morpholino-3-chloro-2- chloromethylpyridine hydrochloride and using corresponding molar proportions of the other reagents in the method of Example 1F gave 2-(4-piperidino-3-bromo-2-pyridylmethylthio)- 5-methoxy-(1H)-benzimidazole, 1.34 g, as an oil.

Example 14

Preparation of 2-(4-piperidino-3-bromo-2-pyridylmethylsulphinyl) -5-methoxy-(1H)-benzimidazole

Substituting 2-(4-piperidino-3-bromo-2-pyridylmethylthio)-5 -methoxy-(1H)-benzimidazole (1.34 g) for 2-(4-morpholino-3-chloro-2 pyridylmethylthio)-5-methoxy-(1H)-benzimidazole and using corresponding molar proportions of the other reagents in the method of Example 2 gave after chromatography (silica gel:2% methanol:chloroform) 2-(4-piperidino-3-bromo-2-pyridylmethyl-sulphinyl)-5- methoxy-(1H)-benzimidazole, 0.62 g, from ether.
$C_{19}H_{21}BrN_4O_2S.H_2O$
Found C 48.53, H 4.58, N 11.75, S 6.80, Br 17.02
Requires C 48.82, H 4.96, N 11.99, S 6.86, Br 17.10

Example 15A

Preparation of 5-bromo-2,3-lutidine-N-oxide

m-Chloroperbenzoic acid (71.42 g) in dichloromethane (1l) was added dropwise to a stirred solution of 5-bromo-2,3- lutidine (70.0g) over 1 hour. After 23 hours the solution was cooled to 15° and ammonia gas bubbled through to give a white precipitate, which was filtered off. The filtrate was washed with 5% aqueous sodium sulphite, dried, and stripped to give 5-bromo-2,3-lutidine-N-oxide, 61.78 g, m.p. 80-2°, from ethyl acetate.

Example 15B

Preparation of 5-bromo-4-chloro-2,3-lutidine

5-bromo-2,3-lutidine-N-oxide (30.31g) was added in portions to phosphorus oxychloride (41.3 ml) at 30-40°. The mixture was heated to 50°, when an exothermic reaction raised the temperature to reflux. After 30 minutes the solution was cooled and poured onto ice to give a white solid, which was filtered off and discarded. The filtrate was extracted with ether then treated with 40% aqueous sodium hydroxide to pH 7, and extracted again with ether. The latter extract was dried ($MgSO_4$) and stripped to give 5-bromo-4-chloro-2,3-lutidine, 12.0 g as a low melting solid.

Example 15C

Preparation of 4-piperidino-5-bromo-2,3-lutidine

Substituting 5-bromo-4-chloro-2,3-lutidine (8.0 g) for 3,4-dichloro-2-hydroxymethyl pyridine and using corresponding molar proportions of the other reagents in the method of Example 1D gave, after chromatography (silica gel, chloroform), 4-piperidino-5-bromo-2,3- lutidine, 7.88 g, as an oil.

Example 15D

Preparation of 4-piperidino-5-bromo-2,3-lutidine-N-oxide

Substituting 4-piperidino-5-bromo-2,3-lutidine (8.3 g) for 4-piperidino-5-bromo-2-picoline and using corresponding molar proportions of the other reagents in the method of Example 11C gave 4-piperidino-5-bromo -2,3-lutidine-N-oxide, 5.31 g, m.p. 103-5°, from ether.

Example 15E

Preparation of 4-piperidino-5-bromo-3-methyl-2-hydroxymethylpyridine

Substituting 4-piperidino-5-bromo-2,3-lutidine-N-oxide (4.71 g) for 4-piperidino-5-bromo-2-picoline-N-oxide and using corresponding molar proportions of the other reagents in the method of Example 17D

gave 4-piperidino-5-bromo-3-methyl-2-hydroxymethylpyridine, 2.34 g, m.p. 80-1°, from pet. ether (60/80).

Example 15F

Preparation of 4-piperidino-5-bromo-3-methyl-2-chloromethylpyridine hydrochloride

Substituting 4-piperidino-5-bromo-3-methyl-2-hydroxymethylpyridine (2.25g) for 4-morpholino-3-chloro-2-hydroxymethylpyridine and using corresponding molar proportions of the other reagents in the method of Example 1E gave 4-piperidino-5-bromo-3-methyl-2-chloromethylpyridine hydrochloride, 2.68 g, m.p. 177-9°, by ether trituration.

Example 15G

Preparation of 2-(4-piperidino-5-bromo-3-methyl-2-pyridylmethylthio) -5-methoxy-(1H)-benzimidazole

Substituting 4-piperidino-5-bromo-3-methyl-2-chloromethylpyridine hydrochloride (2.65 g) for 4-morpholino-3-chloro- 2-chloromethylpyridine hydrochloride and using the corresponding molar proportions of the other reagents in the method of Example 1F gave 2-(4-piperidino-5-bromo-3-methyl-2-pyridylmethylthio)-5-methoxy-(1H) -benzimidazole, 2.97 g, m.p. 90-2° from isopropanol/water.

Example 16A

Preparation of 2-(4-piperidino-5-bromo-3-methyl-2- pyridylmethylsulphinyl)-5-methoxy-(1H)-benzimidazole

Substituting 2-(4-piperidino-5-bromo-3-methyl-2-pyridyl-methylthio -5-methoxy-(1H)-benzimidazole (2.9 g) for 2-(4-morpholino-3-chloro-2 -pyridylmethylthio)-5-methoxy-(1H)-benzimidazole and using corresponding molar proportions of the other reagents in the method of Example 2 gave 2-(4-piperidino-5-bromo-3-methyl-2-pyridyl-methylsulphinyl)-5-methoxy-(1H) -benzimidazole, 1.83 g, m.p. 157-9° (dec), from acetonitrile.
$C_{20}H_{23}BrN_4O_2S$
Found C 51.81, H 5.01, N 11.95, S 6.85, Br 17.18
Requires C 51.84, H 5.00, N 12.09, S 6.92, Br 17.24

Example 17A

Preparation of 2-(4-piperidino-3-chloro-5-methyl-2-pyridylmethylthio) -5-(1,1,2,2-tetrafluoroethoxy)-(1H)-benzimidazole

5N Sodium hydroxide (2 ml) was added to a stirred suspension of 4-piperidino-3-chloro-5-methyl-2-chloromethylpyridine hydrochloride (1.6 g) and 5-(1,1,2,2-tetrafluoroethoxy)-2 -mercaptobenzimidazole (1.5 g) in ethanol at room temperature. After standing over night an additional quantity of sodium hydroxide (1 ml) was added and after a further 1 hour the mixture was evaporated at reduced pressure. The residue was chromatographed (silica gel chloroform/methanol 2%) and crystallised from acetonitrile to give 2-(4-piperidino-3-chloro-5-methyl-2-pyridylmethylthio)-5-(1,1,2,2 -tetrafluoroethoxy)-(1H)- benzimidazole, 1.66 g, m.p. 170-71°.

Example 17B

Preparation of 2-(4-piperidino-3-chloro-5-methyl-2- pyridylmethylsulphinyl)-5-(1,1,2,2-tetrafluoroethoxy)-(1H)- benzimidazole

m-Chloroperbenzoic acid (0.75 g total) in dichloromethane (40 ml) was added to a solution of 2-(4-piperidino-3- chloro-5-methyl-2-pyridylmethylthio)-5-(1,1,2,2-tetrafluoroethoxy)-(1H) -benzimidazole (1.5 g) in dichloromethane (30 ml) at -40 to -50° over a period of 2.5 hours. After a further 0.5 hour ammonia was passed through the solution and the precipitate filtered off and washed well with dichloromethane. The filtrates were evaporated under reduced pressure to give an oil which, on treatment with acetonitrile, gave 2-(4-piperidino-3-chloro-5-methyl-2- pyridylmethylsulphinyl)-5-(1,1,2,2-tetrafluoroethoxy)-(1H)- -benzimidazole, 0.77g, m.p. 155-56° (dec).
$C_{21}H_{21}ClF_4N_4OS$
Found C 49.70, H 4.12, N 10.93, S 6.63
Requires C 49,95, H 4.19, N 11.10, S 6.35

Example 18A

Preparation of 2-(4-piperidino-3-chloro-5-methyl-2- pyridylmethylthio)-4,5-difluoromethylenedioxy-(1H)- benzimidazole

Substituting 4,5-difluoromethylenedioxy-2-mercapto- benzimidazole (1.5 g) for 5-(1,1,2,2-tetrafluoroethoxy)- 2-mercaptobenzimidazole and using corresponding molar proportions of the other reagents in the method of Example 17A gave 2-(4-piperidino-3-chloro 5-methyl-2-pyridyl- methylthio)-4,5-difluoromethylenedioxy-(1H) -benzimidazole, 2.12 g, m.p. 198-99° from acetonitrile.

Example 18B

Preparation of 2-(4-piperidino-3-chloro-5-methyl-2- pyridylmethylsulphinyl)-4,5-difluoromethylenedioxy-(1H)- benzimidazole

Substituting 2-(4-piperidino-3-chloro-5-methyl-2-pyridylmethylthio) -4,5-difluoromethylenedioxy-(1H)-benzimidazole (2 g) for 2-(4-piperidino-3-chloro-5-methyl-2-pyridyl- methylthio)-5-(1,1,2,2-tetrafluoroethoxy)-(1H)-benzimidazole and using corresponding molar proportions of the other reagents in the method of Example 23B gave 2-(4-piperidino- 3-chloro-5-methyl-2-pyridylmethylsulphinyl)-4,5-difluoromethylenedioxy (1H)-benzimidazole, 1.05 g, m.p. 169-70° (dec) from acetonitrile.

Example 19A

Preparation of 4-chloro-5-bromo-2-picoline-N-oxide

A solution of 5-bromo-4-nitro-2-picoline-N-oxide (30.0 g) in dichloromethane (250 ml) was cooled at 10°, and a solution of phosphoryl chloride (35.5 ml) in dichloromethane (200 ml) added over 15 minutes. The mixture was heated under reflux for 5 hours, allowed to cool to ambient temperature, and allowed to stand for 16 hours. After pouring onto ice (300 ml) and stirring for 15 minutes, the mixture was basified to pH 10 using concentrated aqueous sodium hydroxide. The organic phase was separated off, and the aqueous phase further extracted with chloroform (2x100 ml). The combined organic phases were dried ($K_2CO_3$) and stripped to a solid, which was triturated with petroleum ether (40-60), filtered, washed and dried to yield 4-chloro-5-bromo-2-picoline-N-oxide, 24.08 g, m.p. 121-4°C.

Example 19B

Preparation of 4-chloro-5-bromo-2-hydroxymethylpyridine

A solution of 4-chloro-5-bromo-2-picoline-N-oxide (23.9 g) in dichloromethane (250 ml) was cooled to 10° and trifluoroacetic anhydride (25 ml) added over 20minutes. The mixture was allowed to warm to ambient temperature, and stand for 7 days. After cooling to 10°, methanol (100 ml) was added, and the solution stripped. The residue was treated with water (150 ml), basified with saturated aqueous sodium carbonate to pH 10, and extracted with ethyl acetate (2x150 ml). The combined extracts were dried ($MgSO_4$), stripped, and triturated with ether to give 4-chloro-5-bromo-2-hydroxymethylpyridine, 16.58 g, m.p. 109-10°.

Example 19C

Preparation of 4-dimethylamino-5-bromo-2-hydroxymethylpyridine

Substituting 33% w/w dimethylamine in ethanol (15 ml) for morpholine and 4-chloro-5-bromo-2-hydroxymethylpyridine (5 g) for 3,4-dichloro-2-hydroxymethylpyridine and using corresponding molar proportions of the other reagents in the method of Example 1D gave after chromatography (silica, chloroform:methanol, 98:2) 4-dimethylamino-5 -bromo-2-hydroxymethylpyridine, 3.62 g, as an oil.

Example 19D

Preparation of 4-dimethylamino-5-bromo-2-chloromethyl-pyridine hydrochloride

Substituting 4-dimethylamino-5-bromo-2-hydroxymethylpyridine (3.42 g) for 4-morpholino-3-chloro-2-hydroxy-methylpyridine and using corresponding molar proportions of the other reagents in the method of Example 1E gave 4-dimethylamino-5-bromo-2-chloromethylpyridine hydrochloride, 4.11 g, m.p. 184-5° (dec.).

Example 19E

Preparation of 2-(4-dimethylamino-5-bromo-2-pyridyl-methylthio)-5 -methoxy-(1H)-benzimidazole

Substituting 4-dimethylamino-5-bromo-2-chloromethylpyridine hydrochloride (3.96 g) for 4-morpholino-3- chloro-2-chloromethylpyridine hydrochloride and using corresponding molar proportions of the other reagents in the method of Example 1F gave after chromatography 2-(4-dimethylamino-5-bromo-2-pyridylmethylthio)-5-methoxy (1H)-benzimidazole, 5.23 g, as a foam.

Example 19F

Preparation of 2-(4-dimethylamino-5-bromo-2-pyridylmethylsulphinyl) -5-methoxy-(1H)-benzimidazole

Substituting 2-(4-dimethylamino-5-bromo-2-pyridylmethylthio)-5 -methoxy-(1H)-benzimidazole (5.02 g) for 2-(4-morpholino-3-chloro-2 pyridylmethylthio)-5-methoxy-(1H)-benzimidazole and using corresponding molar proportions of the other reagents in the method of Example 2 gave 2-(4-dimethylamino-5-bromo-2-pyridylmethylsulphinyl)-5-methoxy-(1H) -benzimidazole, 3.8 g.
$C_{16}H_{17}BrN_4O_2S$
Found C 47.18, H 4.14, N 13.60, S 7.81, Br 19.34
Requires C 46.95, H 4.19, N 13.69, S 7.83, Br 19.52

Example A

A tablet for oral administration is prepared by combining

| | Mg/Tablet |
|---|---|
| Compound of structure (I) | 100 |
| Mannitol | 153 |
| Starch | 33 |
| crospovidone | 12 |
| microcrystalline cellulose | 30 |
| magnesium stearate | 2 |
| | 330 mg |

into a 9 mm tablet. If the active ingredient is a compound of structure (I) in which n is 1 then the tablet is provided with an enteric coating.

Example B

A pellet formulation for oral administration may be prepared by formulating the following into pellets by standard techniques

| | %w:w |
|---|---|
| Compound of structure (I) | 80 |
| microcrystalline cellulose | 10 |
| sodium carboxymethylcellulose | 2 |
| lactose | 8 |

If the active ingredient is a compound of structure (I) in which n is 1, the pellets are first enteric coated before being filled into hard gelatin capsules.

Example C

An injection for parenteral administration is prepared by combining

| | | %w:w |
|---|---|---|
| Compound of example 1 | | 1–5 |
| polypropylene glycol | | 40 |
| ethanol | | 10 |
| water for injection EP | to | 100 |

The solution is then sterilised and sealed into 2 ml and 5 ml ampoules and vials.

Example D

A reconstitutable lyophilisate for parenteral administration is prepared from:

| | %w:w |
|---|---|
| Compound of structure (I) as a salt | 1–5 |
| Mannitol | 15 |
| NaCl | sufficient to make reconstituted solution isotonic |
| water | to 100 |

The solution is sterilised by aseptic filtration, 5 ml portions dispensed into 15 ml vials and the solution lyophilised. The lyophilisate can be reconstituted with 10 ml $H_2O$.

Biological Data

A. $K^+$ Stimulated ATPase Activity.

The effects of a single high concentration (1mM) of a compound of structure (I) on $H^+$-$K^+$ ATPase were determined at pH 6.1 and pH 7.4. Preferred compounds of structure (I) were also tested over a range of concentrations to determine $IC_{50}$ values at pH 6.1 and 7.4.

(i) Preparation of Lyophilised Gastric Vesicles ($H^+$-$K^+$-ATPase)

$H^+$-$K^+$-ATPase was prepared from the lyophilised gastric vesicles of pig fundic mucosa after the method of Saccomani et. al. (Biochem and Biophys. Acta., 465, 311, 1977).

(ii) $K^+$ Stimulated ATPase Activity

Compounds of structure (I) were pre-incubated with $H^+$-$K^+$-ATPase preparation 30μg protein/ml from (i) in 10 mM Pipes/Tris buffer pH 6.1 and pH 7.4.

After 30 min at 37° the pre-incubation was diluted 5-fold with assay buffer to start the ATPase reaction. The conditions in the assay are 100mM Pipes/Tris, 2mM $MgCl_2$, 10mM KCl, 5ug/ml nigericin, 2mM $Na_2$ATP, pH 7.0. After an incubation at 37° for 15 min the inorganic phosphate released was determined by the method of Yoda & Hokin (Biochem. Biophys. Res. Com. 40, 880, 1970). Nigericin was dissolved in methanol, which at the final concentration of 0.5%, did not affect the enzyme activity.

The effect of the same concentration of compound of structure (I) (pre-incubated with $H^+$-$K^+$-ATPase preparation at pH 7.4 as described above) on the recovery of 100nmole of inorganic phosphate was also determined.

Compounds of structure (I) were initially dissolved in dimethyl sulphoxide, polyethylene glycol (Type 400) or Pipes/Tris buffer. None of these solvents affects $K^+$-ATPase activity at the concentrations used.

All data refer to the concentration of compound present in the pre-incubation before dilution with assay buffer.

(iii) Results

The compounds of Examples 2, 4, 6, 8, 10, 12, 14, 16, 17, 18 und 19 were all found to inhibit potassium stimulated ATPase activity in the above preparations at pH 6.1 and 7.4.

B. Aminopyrine (AP) accumulation in intact gastric glands.

The effect of a single concentration (100µm) of a compound of structure (I) on dibutyryl cAMP stimulated AP metabolism in rabbit intact gastric glands was determined. Preferred compounds of structure (I) were tested over a range of concentrations to determine the $IC_{50}$ value.

(i) Preparation of intact gastric glands.

Intact gastric glands were prepared from New Zealand white rabbits by the method of Berghindh et al. (Acta. Physio. Scand. 96, 150, 1976). Gastric mucosal scrapings were digested at 37°C for 45-60 min. with Collagenase (100 U, Type 1, Sigma), and glands harvested by coarse filtration and sedimentation.

(ii) AP accumulation

Test compound was incubated with glands and 300 µM dibutyryl cAMP for 30 minutes at 37°C. Incubating medium contained 132.5 mM NaCl, 5.4 mM KCl, 1.0 mM $NaH_2PO_4$, 5.0 mM $Na_2HPO_4$, 1.2 mM $MgSO_4$, 1.0 mM $CaCl_2$, 11.1 mM glucose, 2.0 mg/ml rabbit albumin, 10µg/ml phenol red, approximately 0.3 µM[$^{14}$C] aminopyrine (110 mCi/mmole), pH 7.4.

After incubation, glands were centrifuged and the supernatant removed. The glands were dried, weighed and dissolved in NaOH. The distribution of radioactivity between the supernatant and glands is then used to calculate the AP ratio after the method of Berglindh et al. (Acta. Physiol. Scand. 97, 401, 1976).

The $IC_{50}$ value is the amount of compound required to inhibit the histamine stimulated accumulation of aminopyrine by 50%.

(iii) Results

| Compound of Example | $IC_{50}$ (µM) |
|---|---|
| 2 | 11.0 |
| 4 | 16.3 |
| 6 | 4.2 |
| 8 | 2.0 |
| 10 | 3.7 |
| 12 | 1.6 |
| 14 | 1.3 |
| 16 | 9.2 |
| 17 | 1.9 |

C. Rat : Lumen perfused stomach (histamine stimulated gastric acid secretion).

Using a modification of the procedure described by Ghosh and Schild (Br. J. Pharmacology, 13, 54, 1958), $ED_{50}$ values after either intraduodenal (i.d) or intravenous (i.v) administration were obtained as follows:

| Compound of Example | Route of Administration | $ED_{50}$ µmol/kg |
|---|---|---|
| 2 | i.v. | 1.17 |
| 4 | i.d. | 1.94 |
| 6 | i.d. | 2.38 |
| 10 | i.d. | 1.46 |
| 12 | i.v. | 0.85 |
| 14 | i.v. | 0.86 |
| 16 | i.d. | 4.55 |

No overt signs of toxicity were observed in any of the foregoing tests.

**Claims for the Contracting States BE CH DE FR GB IT LI LU NL SE:**

1. A compound of structure (I)

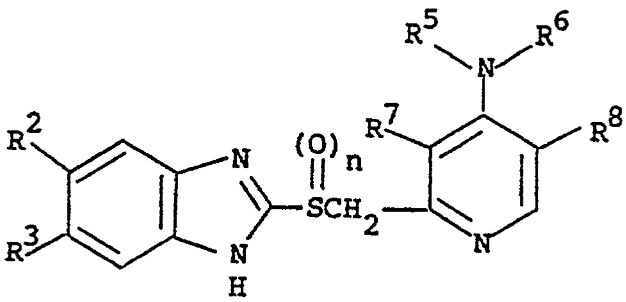

(I)

in which,
$R^2$ and $R^3$ are the same or different and are each hydrogen, $C_{1-6}$alkyl, $C_{1-6}$alkoxy, $RCF_2O$, an ethoxy group substituted by 3 to 5 fluorine atoms, or $R^2$ and $R^3$ together form a group $-O(CR_2)_m-$;
R is hydrogen or fluorine;
m is 1 or 2;
n is 0 or 1;
$R^5$ and $R^6$ are the same or different and are each $C_{1-6}$alkyl or $R^5$ and $R^6$ together with the nitrogen atom to which they are attached form a piperidino or morpholino-group; and
one of $R^7$ and $R^8$ is halogen, and the other is hydrogen, halogen or $C_{1-6}$alkyl;
or a pharmaceutically acceptable salt thereof.

2. A compound as claimed in claim 1 in which $R^5$ and $R^6$ together with the nitrogen atom to which they are attached form a piperidino or morpholino group.

3. A compound as claimed in claim 1 or claim 2 in which n is 1.

4. A compound as claimed in claim 1 which is:
2-(3-chloro-4-piperidino-2-pyridylmethylsulphinyl)-5-methoxy-(1H)-benzimidazole,
2-(3-chloro-5-methyl-4-piperidino-2-pyridylmethylsulphinyl)-5-methoxy-(1H)-benzimidazole,
2-(3-chloro-4-morpholino-2-pyridylmethylsulphinyl)-5-methoxy-(1H)-benzimidazole,
2-(3-chloro-5-methyl-4-morpholino-2-pyridylmethylsulphinyl)-5-methoxy-(1H)-benzimidazole.

5. A compound as claimed in claim 1 which is:
2-(5-bromo-4-piperidino-2-pyridylmethylsulphinyl)-5-methoxy-(1H)-benzimidazole,
2-(3-bromo-5-piperidino-2-pyridylmethylsulphinyl)-5-methoxy-(1H)-benzimidazole,
2-(3-methyl-5-bromo-4-piperidino-2-pyridylmethylsulphinyl)-5-methoxy-(1H)-benzimidazole,
2-(4-dimethylamino-3-chloro-5-methyl-2-pyridylmethylsulphinyl)-5-methoxy-1H-benzimidazole.

6. A pharmaceutical composition comprising a compound as claimed in claim 1 in association with a pharmaceutically acceptable carrier.

7. A pharmaceutical composition as claimed in claim 6, in which n is 1.

8. A pharmaceutical composition as claimed in claim 7, in a form suitable for oral administration.

9. A pharmaceutical composition as claimed in claim 8, provided with an enteric coating.

10. A compound as claimed in any one of claims 1 to 5 for use as a therapeutic agent.

11. A compound as claimed in any one of claims 1 to 5 for use in the treatment of gastro-intestinal diseases caused or exacerbated by gastric acidity.

12. A process for the preparation of a compound as claimed in claim 1, which comprises
a) reacting a compound of structure (II)

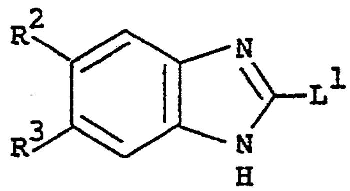

(II)

with a compound of structure (III)

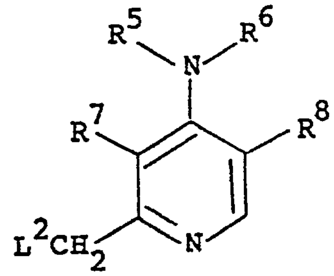

(III)

in which $R^2$, $R^3$ and $R^5$ to $R^8$ are as described for structure (I), one of $L^1$ and $L^2$ is SH and the other is a leaving group displaceable by a mercaptan;
(b) reacting a compound of structure (IV)

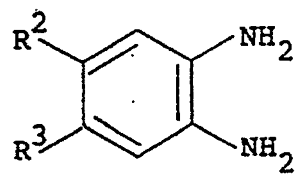

(IV)

in which $R^2$ and $R^3$ are as described for structure (I), with a compound of structure (V)

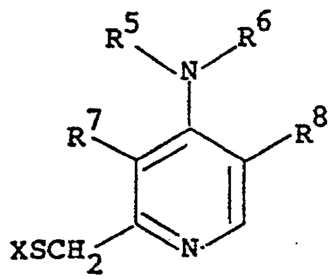

(V)

in which $R^5$ to $R^8$ are as described for structure (I), X is $CO_2H$ or $CSX^1$ and $X^1$ is halogen or $C_{1-4}$alkoxy;
(c) reacting a compound of structure (VI)

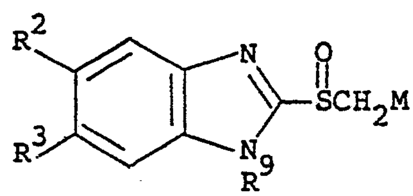

(VI)

in which $R^2$ and $R^3$ are as described for structure (I) in claim 1, $R^9$ is hydrogen or a protecting group and M is an alkali metal atom, with a compound of structure (VII)

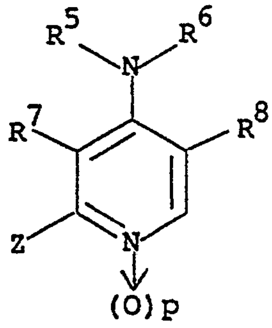

(VII)

in which $R^5$ to $R^8$ are as described for structure (I), Z is a leaving group and p is 0 or 1;
(d) reacting a compound of structure (VIII)

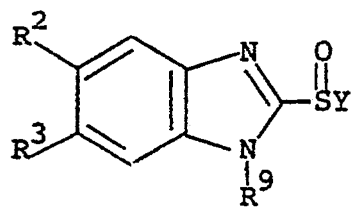

(VIII)

in which $R^2$ and $R^3$ are as described for structure (I), $R^9$ is hydrogen or a protecting group and Y is a leaving group, with a compound of structure (IX)

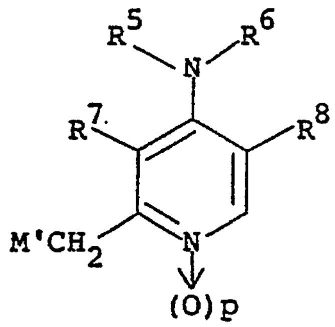

(IX)

in which $R^5$ to $R^8$ are as described for structure (I), p is 0 or 1 and M' is the equivalent of a metal atom, and, optionally where desired
(i) oxidising a compound of structure (I) in which n is 0 so formed to a compound of structure (I) in which n is 1;
(ii) reducing a compound of structure (I) so formed in which n is 1 to a compound of structure (I) in which n is 0;
(iii) forming a pharmaceutically acceptable salt.

13. A compound of structure (III)

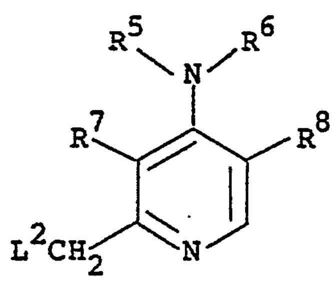

(III)

in which $R^5$ to $R^8$ are as described for structure (I) and $L^2$ is SH or a leaving group displaceable by mercaptan.

14. A compound of structure (V)

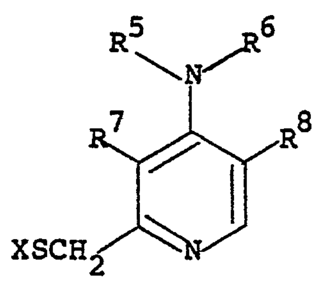

(V)

in which $R^5$ to $R^8$ are as described for structure (I), and X is $CO_2H$ or $CSX^1$ and $X^1$ is halogen or $C_{1-4}$alkoxy.

**Claims for the Contracting State AT:**

1. A process for the preparation of a compound of structure (I)

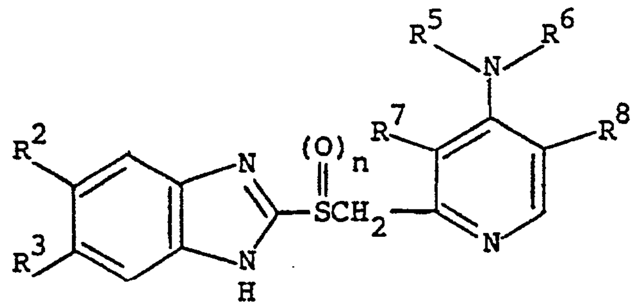

(I)

in which,

$R^2$ and $R^3$ are the same or different and are each hydrogen, $C_{1-6}$alkyl, $C_{1-6}$alkoxy, $RCF_2O$, an ethoxy group substituted by 3 to 5 fluorine atoms, or $R^2$ and $R^3$ together form a group $-O(CR_2)_mO-$;
R is hydrogen or fluorine;
m is 1 or 2;
n is 0 or 1;
$R^5$ and $R^6$ are the same or different and are each $C_{1-6}$alkyl or $R^5$ and $R^6$ together with the nitrogen atom to which they are attached form a piperidino or morpholino-group; and
one of $R^7$ and $R^8$ is halogen, and the other is hydrogen, halogen or $C_{1-6}$alkyl;
or a pharmaceutically acceptable salt thereof, which comprises
a) reacting a compound of structure (II)

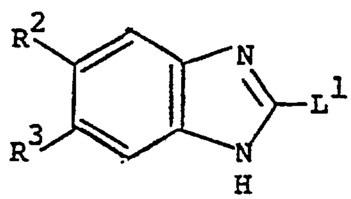

(II)

with a compound of structure (III)

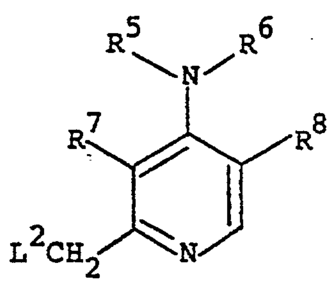

(III)

in which $R^2$, $R^3$ and $R^5$ to $R^8$ are as described for structure (I), one of $L^1$ and $L^2$ is SH and the other is a leaving group displaceable by a mercaptan;
b) reacting a compound of structure (IV)

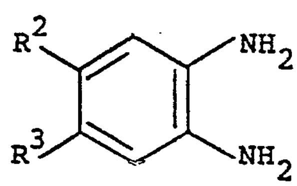

(IV)

in which $R^2$ and $R^3$ are as described for structure (I), with a compound of structure (V)

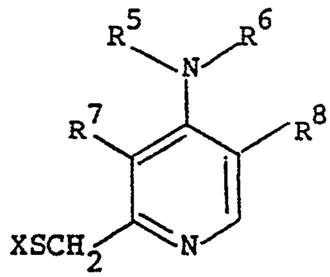

(V)

in which $R^5$ to R8 are as described for structure (I), X is $CO_2H$ or $CSX^1$ and $X^1$ is halogen or $C_{1-4}$alkoxy;

(c) reacting a compound of structure (VI)

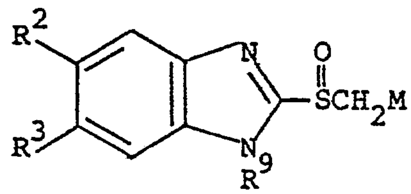

(VI)

in which $R^2$ and $R^3$ are as described for structure (I), $R^9$ is hydrogen or a protecting group and M is an alkali metal atom, with a compound of structure (VII)

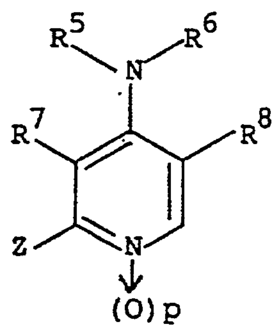

(VII)

in which $R^5$ and $R^6$ are as described for structure (I), Z is a leaving group and p is 0 or 1;

(d) reacting a compound of structure (VIII)

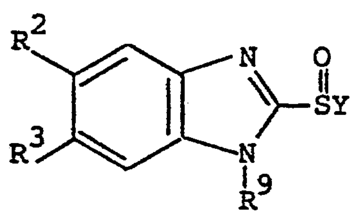

(VIII)

in which $R^2$ and $R^3$ are as described for structure (I), $R^9$ is hydrogen or a protecting group and Y is a leaving group, with a compound of structure (IX)

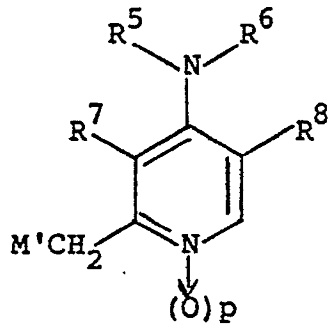

(IX)

in which $R^5$ to $R^8$ are as described for structure (I), p is 0 or 1 and M' is the equivalent of a metal atom, and, optionally where desired,
(i) oxidising a compound of structure (I) in which n is 0 so formed to a compound of structure (I) in which n is 1;
(ii) reducing a compound of structure (I) so formed in which n is 1 to a compound of structure (I) in which n is 0;
(iii) forming a pharmaceutically acceptable salt.

2. A process as claimed in Claim 1 in which $L^1$ is SH and $L^2$ is a leaving group.
3. A process as claimed in Claim 2 in which $L^2$ is chlorine.
4. A process as claimed in any one of claims 1 to 3 when used to prepare a compound as claimed in claim 1 in which n is 1.
5. A process as claimed in any one of Claims 1 to 4 when used to prepare a compound of claim 1 in which $R^5$ and $R^6$ together with the nigrogen atom to which they are attached form a piperidino or morpholino-group.
6. A process as claimed in any one of claims 1 to 5 when used to prepare
2-(3-chloro-4-piperidino-2-pyridylmethylsulphinyl)-5-methoxy-(1H)-benzimidazole,
2-(3-chloro-5-methyl-4-piperidino-2-pyridylmethylsulphinyl)-5-methoxy-(1H)-benzimidazole,
2-(3-chloro-4-morpholino-3-pyridylmethylsulphinyl)-5-methoxy-(1H)-benzimidazole,
2-(3-chloro-5-methyl-4-morpholino-2-pyridylmethylsulphinyl)-5-methoxy-(1H)-benzimidazole,
or a pharmaceutically acceptable salt thereof.
7. A process as claimed in any one of claims 1 to 5 when used to prepare:
2-(3-methyl-5-bromo-4-piperidino-2-pyridylmethylsulphinyl)-5-methoxy-(1H)-benzimidazole.
8. A process for preparing a pharmaceutical composition which comprises bringing into association a compound as claimed in claim 1 and a pharmaceutically acceptable carrier.
9. A process as claimed in Claim 8 in which n is 1.
10. A process as claimed in Claim 9 in which the composition is further provided with an enteric coating.

**Patentansprüche für die Vertragsstaaten BE CH DE FR GB IT LI LU NL SE:**

1. Eine Verbindung der Struktur (I)

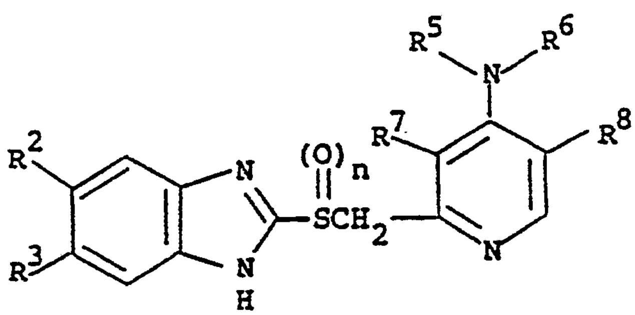

(I)

in der
$R^2$ und $R^3$ gleich oder verschieden sind und jeweils Wasserstoff, $C_{1-6}$-Alkyl, $C_{1-6}$-Alkoxy, $RCF_2O$, eine durch 3 bis 5 Fluoratome substituierte Äthoxygruppe bedeuten oder $R^2$ und $R^3$ zusammen einen Rest $-O(CR_2)_mO-$ bilden;
R Wasserstoff oder Fluor darstellt;
m 1 oder 2 ist;
n 0 oder 1 ist;
$R^5$ und $R^6$ gleich oder verschieden sind und jeweils $C_{1-6}$-Alkyl bedeuten oder $R^5$ und $R^6$ zusammen mit dem Stickstoffatom, an das sie gebunden sind, eine Piperidino- oder Morpholinogruppe bilden; und

einer der Reste $R^7$ und $R^8$ Halogen ist und der andere Wasserstoff, Halogen oder $C_{1-6}$-Alkyl bedeutet;
oder ein pharmazeutisch verträgliches Salz davon.

2. Verbindung nach Anspruch 1, in der $R^5$ und $R^6$ zusammen mit dem Stickstoffatom, an das sie gebunden sind, eine Piperidino- oder Morpholinogruppe bilden.

3. Verbindung nach Anspruch 1 oder Anspruch 2, in der n 1 ist.

4. Verbindung nach Anspruch 1, nämlich:
2-(3-Chlor-4-piperidino-2-pyridylmethylsulfinyl)-5-methoxy-(1H)-benzimidazol,
2-(3-Chlor-5-methyl-4-piperidino-2-pyridylmethylsulfinyl)-5-methoxy-(1H)-benzimidazol,
2-(3-Chlor-4-morpholino-2-pyridylmethylsulfinyl)-5-methoxy-(1H)-benzimidazol,
2-(3-Chlor-5-methyl-4-morpholino-2-pyridylmethylsulfinyl)-5-methoxy-(1H)-benzimidazol.

5. Verbindung nach Anspruch 1, nämlich
2-(5-Brom-4-piperidino-2-pyridylmethylsulfinyl)-5-methoxy-(1H)-benzimidazol.
2-(3-Brom-4-piperidino-2-pyridylmethylsulfinyl)-5-methoxy-(1H)-benzimidazol.
2-(3-Methyl-5-brom-4-piperidino-2-pyridylmethylsulfinyl)-5-methoxy-(1H)-benzimidazol
2-(4-Dimethylamino-3-chlor-5-methyl-2-pyridylmethylsulfinyl)-5-methoxy-1H-benzimidazol.

6. Arzneimittel, umfassend eine Verbindung nach Anspruch 1 in Verbindung mit einem pharmazeutisch verträglichen Träger.

7. Arzneimittel nach Anspruch 6, in dem n 1 ist.

8. Arzneimittel nach Anspruch 7, in einer zur oralen Verabreichung geeigneten Form.

9. Arzneimittel nach Anspruch 8, versehen mit einer enterischen Beschichtung.

10. Verbindung nach einem der Ansprüche 1 bis 5 zur Verwendung als therapeutischer Wirkstoff.

11. Verbindung nach einem der Ansprüche 1 bis 5 zur Verwendung bei der Behandlung von Gastro-Intestinal-Erkrankungen, die durch Magensäure verursacht oder verschlimmert werden.

12. Verfahren zur Herstellung einer Verbindung nach Anspruch 1, umfassend
a) Umsetzung einer Verbindung der Struktur (II)

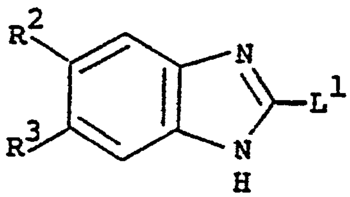

(II)

mit einer Verbindung der Struktur (III)

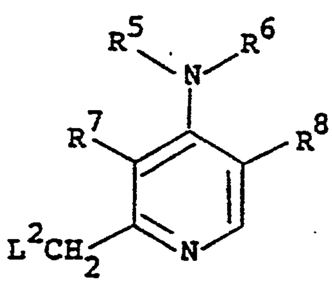

(III)

in der $R^2$, $R^3$ und $R^5$ bis $R^8$ die für die Struktur (I) beschriebene Bedeutung haben, einer der Reste $L^1$ und $L^2$ die Bedeutung SH hat und der andere eine durch ein Mercaptan verdrängbare austretende Gruppe ist;
b) Umsetzung einer Verbindung der Struktur (IV)

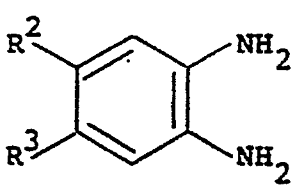

(IV)

in der $R^2$ und $R^3$ die für Struktur (I) beschriebene Bedeutung haben, mit einer Verbindung der Struktur (V)

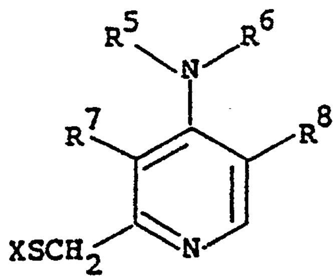

(V)

in der $R^5$ bis $R^8$ die für Struktur (I) beschriebene Bedeutung haben, X die Bedeutung $CO_2H$ oder $CSX^1$ aufweist und $X^1$ Halogen oder $C_{1-4}$-Alkoxy ist;

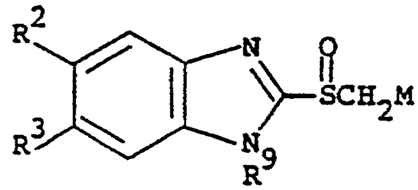

(VI)

in der $R^2$ und $R^3$ die für Struktur (I) im Anspruch 1 beschriebene Bedeutung haben, $R^9$ Wasserstoff oder eine Schutzgruppe darstellt und M ein Alkalimetallatom ist mit einer Verbindung der Struktur (VII)

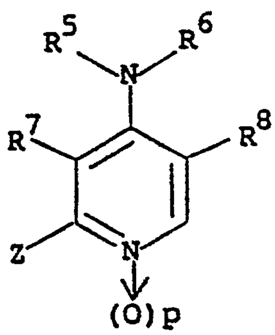

(VII)

in der $R^5$ bis $R^8$ die für Struktur (I) angegebene Bedeutung haben, Z eine austretende Gruppe darstellt und p 0 oder 1 ist;
d) Umsetzung einer Verbindung der Struktur (VIII)

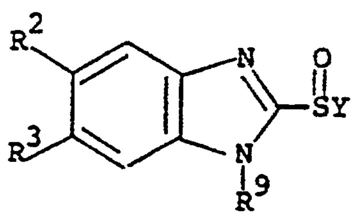

(VIII)

in der $R^2$ und $R^3$ die für Struktur (I) angegebene Bedeutung haben, $R^9$ Wasserstoff oder eine Schutzgruppe ist und Y eine austretende Gruppe bedeutet, mit einer Verbindung der Struktur (IX)

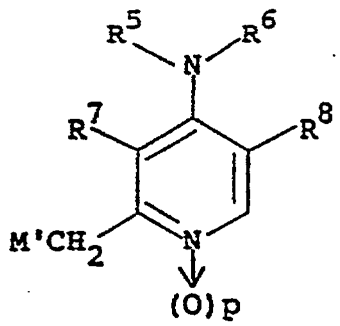

(IX)

in der $R^5$ bis $R^8$ die für Struktur (I) angegebene Bedeutung haben, p 0 oder 1 ist und m' ein Äquivalent eines Metallatoms bedeutet,
und, gegebenenfalls, wenn erwünscht,
(i) Oxidieren einer so erhaltenen Verbindung der Struktur (I), in der n 0 ist, zu einer Verbindung der Struktur (I), in der n 1 ist,
(ii) Reduzieren einer so erhaltenen Verbindung der Struktur (I), in der n 1 ist, zu einer Verbindung der Struktur (I), in der n 0 ist;
(iii) Erzeugen eines pharmazeutisch verträglichen Salzes.

13. Eine Verbindung der Struktur (III)

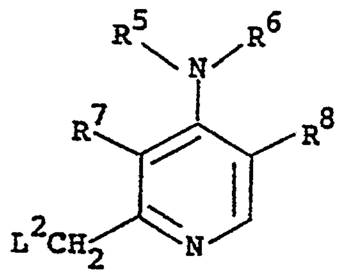

(III)

in der $R^5$ bis $R^8$ die für Struktur (I) beschriebene Bedeutung haben und $L^2$ die Bedeutung SH hat oder eine durch Mercaptan verdrängbare austretende Gruppe ist.

14. Verbindung der Struktur (V)

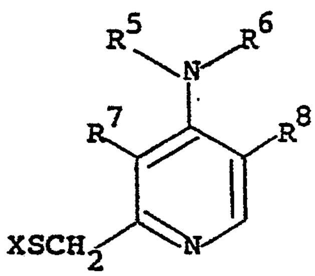

(V)

in der $R^5$ bis $R^8$ die für Struktur (I) beschriebene Bedeutung haben, X die Bedeutung $CO_2H$ oder $CSX^1$ hat und $X^1$ Halogen oder $C_{1-4}$-Alkoxy ist.

**Patentansprüche für den Vertragsstaat AT:**

1. Verfahren zur Herstellung einer Verbindung der Struktur (I)

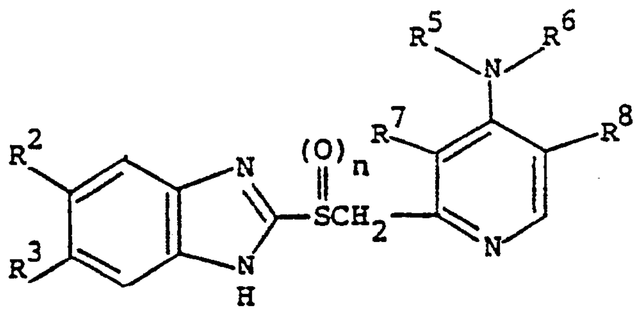

(I)

in der

$R^2$ und $R^3$ gleich oder verschieden sind und jeweils Wasserstoff, $C_{1-6}$-Alkyl, $C_{1-6}$-Alkoxy, $RCF_2O$, eine durch 3 bis 5 Fluoratome substituierte Äthoxygruppe bedeuten oder $R^2$ und $R^3$ zusammen einen Rest $-O(CR_2)_mO-$ bilden;

R Wasserstoff oder Fluor darstellt;

m 1 oder 2 ist;

n 0 oder 1 ist;

$R^5$ und $R^6$ gleich oder verschieden sind und jeweils $C_{1-6}$-Alkyl bedeuten oder $R^5$ und $R^6$ zusammen mit dem Stickstoffatom, an das sie gebunden sind, eine Piperidino- oder Morpholinogruppe bilden; und einer der Reste $R^7$ und $R^8$ Halogen ist und der andere Wasserstoff, Halogen oder $C_{1-6}$-Alkyl bedeutet;

oder eines pharmazeutisch verträglichen Salzes davon,

umfassend

a) Umsetzung einer Verbindung der Struktur (II)

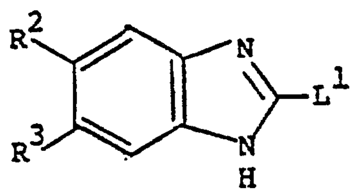

(II)

mit einer Verbindung der Struktur (III)

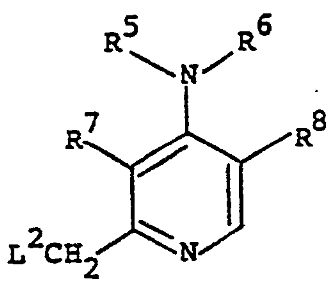

(III)

in der $R^2$, $R^3$ und $R^5$ bis $R^8$ die für die Struktur (I) beschriebene Bedeutung haben, einer der Reste $L^1$ und $L^2$ die Bedeutung SH hat und der andere eine durch ein Mercaptan verdrängbare austretende Gruppe ist;

b) Umsetzung einer Verbindung der Struktur (IV)

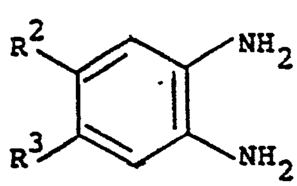

(IV)

in der $R^2$ und $R^3$ die für Struktur (I) beschriebene Bedeutung haben, mit einer Verbindung der Struktur (V)

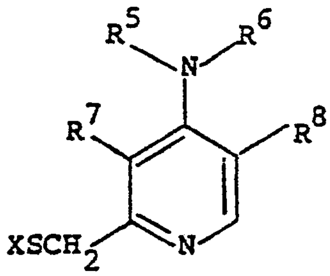

(V)

in der $R^5$ bis $R^8$ die für Struktur (I) beschriebene Bedeutung haben, X die Bedeutung $CO_2H$ oder $CSX^1$ aufweist und $X^1$ Halogen oder $C_{1-4}$-Alkoxy ist;
c) Umsetzung einer Verbindung der Struktur (VI)

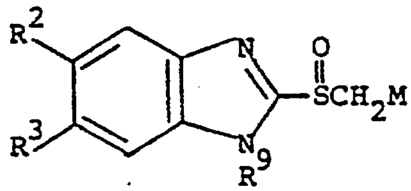

(VI)

in der $R^2$ und $R^3$ die für Struktur (I) beschriebene Bedeutung haben, $R^9$ Wasserstoff oder eine Schutzgruppe darstellt und M ein Alkalimetallatom ist, mit einer Verbindung der Struktur (VII)

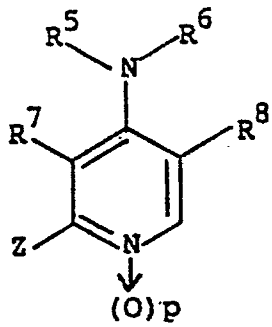

(VII)

in der $R^5$ bis $R^8$ die für Struktur (I) angegebene Bedeutung haben, Z eine austretende Gruppe darstellt und p 0 oder 1 ist;
d) Umsetzung einer Verbindung der Struktur (VIII)

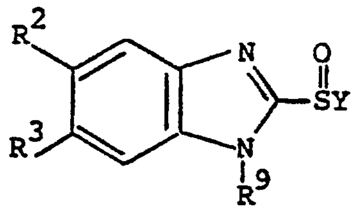

(VIII)

in der $R^2$ und $R^3$ die für Struktur (I) angegebene Bedeutung haben, $R^9$ Wasserstoff oder eine Schutzgruppe ist und Y eine austretende Gruppe bedeutet, mit einer Verbindung der Struktur (IX)

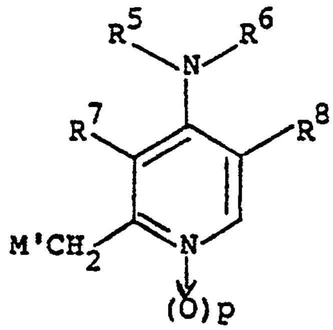

(IX)

in der $R^5$ bis $R^8$ die für Struktur (I) angegebene Bedeutung haben, p 0 oder 1 ist und m' ein Äquivalent eines Metallatoms bedeutet,

und, gegebenenfalls, wenn erwünscht,

(i) Oxidieren einer so erhaltenen Verbindung der Struktur (I), in der n 0 ist, zu einer Verbindung der Strukur (I), in der n 1 ist;

(ii) Reduzieren einer so erhaltenen Verbindung der Struktur (I), in der n 1 ist, zu einer Verbindung der Struktur (I), in der n 0 ist;

(iii) Erzeugen eines pharmazeutisch verträglichen Salzes.

2. Verfahren nach Anspruch 1, wobei $L^1$ die Bedeutung SH hat und und $L^2$ eine austretende Gruppe ist.

3. Verfahren nach Anspruch 2, wobei $L^2$ Chlor ist.

4. Verfahren nach einem der Ansprüche 1 bis 3, soweit angewendet zur Herstellung einer Verbindung nach Anspruch 1, in der n 1 ist.

5. Verfahren nach einem der Ansprüche 1 bis 4, soweit angewendet zur Herstellung einer Verbindung nach Anspruch 1, in der $R^5$ und $R^6$ zusammen mit dem Stickstoffatom, an das sie gebunden sind, eine Piperidino- oder Morpholinogruppe bilden.

6. Verfahren nach einem der Ansprüche 1 bis 5, soweit angewendet zur Herstellung von
2-(3-Chlor-4-piperidino-2-pyridylmethylsulfinyl)-5-methoxy-(1H)-benzimidazol,
2-(3-Chlor-5-methyl-4-piperidino-2-pyridylmethylsulfinyl)-5-methoxy-(1H)-benzimidazol,
2-(3-Chlor-4-morpholino-2-pyridylmethylsulfinyl)-5-methoxy-(1H)-benzimidazol,
2-(3-Chlor-5-methyl-4-morpholino-2-pyridylmethylsulfinyl)-5-methoxy-(1H)-benzimidazol,
oder eines pharmazeutisch verträglichen Salzes davon.

7. Verfahren nach einem der Ansprüche 1 bis 5, sofern angewendet zur Herstellung von:
2-(3-Methyl-5-brom-4-piperidino-2-pyridylmethylsulfinyl)-5-methoxy-(1H)-benzimidazol.

8. Verfahren zur Herstellung eines Arzneimittels, umfassend das Zusammenbringen einer Verbindung nach Anspruch 1 und eines pharmazeutisch verträglichen Trägers.

9. Verfahren nach Anspruch 8, wobei n 1 ist.

10. Verfahren nach Anspruch 9, wobei die Zusammensetzung ferner mit einer enterischen Beschichtung versehen wird.

**Revendications pour les Etats contractants BE CH DE FR GB IT LI LU NL SE:**

1. Composé de structure (I)

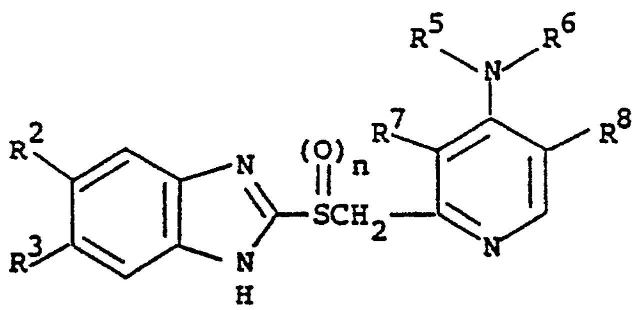

(I)

dans laquelle

$R^2$ et $R^3$ sont identiques ou différents et sont, chacun, de l'hydrogène, un alkyle en $C_{1-6}$, un alkoxy en $C_{1-6}$, $RCF_2O$, un groupement éthoxy substitué par 3 à 5 atomes de fluor ou $R^2$ et $R^3$ forment ensemble un groupement $-O(CR_2)_mO-$;

R est de l'hydrogène ou du fluor;

m est 1 ou 2:

n est 0 ou 1;
$R^5$ et $R^6$ sont identiques ou différents et sont chachun un alkyle en $C_{1-6}$ ou $R^5$ et $R^6$ forment ensemble avec l'atome d'azote auquel ils sont attachés un groupement pipéridino ou morpholino et un des $R^7$ et $R^8$ est un halogène et l'autre est de l'hydrogène, un halogène ou un alkyle en $C_{1-6}$;
ou un sel pharmaceutiquement acceptable de celui-ci.

2. Composé tel que revendiqué dans la revendication 1 dans laquelle $R^5$ et $R^6$ forment ensemble avec l'atome d'azote auquel ils sont attachés un groupement pipéridino ou morpholino.

3. Composé tel que revendiqué dans la revendication 1 ou la revendication 2 dans laquelle n est 1.

4. Composé tel que revendiqué dans la revendication 1 qui est:
2-(3-chloro-4-pipéridino-2-pyridylméthylsulfinyl)-5-méthoxy-(1H)-benzimidazole,
2-(3-chloro-5-méthyl-4-pipéridino-2-pyridylméthylsulfinyl)-5-méthoxy-(1H)-benzimidazole,
2-(3-chloro-4-morpholino-2-pyridylméthylsulfinyl)-5-méthoxy-(1H)-benzimidazole.

5. Composé tel que revendiqué dans la revendication 1 qui est:
2-(5-bromo-4-pipéridino-2-pyridylméthylsulfinyl)-5-méthoxy-(1H)-benzimidazole,
2-(3-bromo-4-pipéridino-2-pyridylméthylsulfinyl)-5-méthoxy-(1H)-benzimidazole,
2-(3-méthyl-5-bromo-4-pipéridino-2-pyridylméthylsulfinyl)-5-méthoxy-(1H)-benzimidazole,
2-(4-diméthylamino-3-chloro-5-méthyl-2-pyridylméthylsulfinyl)-5-méthoxy-(1H)-benzimidazole
2-(3-chloro-5-méthyl-4-morpholino-2-pyridylméthylsulfinyl)-5-méthoxy-(1H)-benzimidazole.

6. Composition pharmaceutique comprenant un composé tel que revendiqué dans la revendication 1 en association avec un support pharmaceutiquement acceptable.

7. Composition pharmaceutique telle que revendiquée dans la revendication 6 dans laquelle n est 1.

8. Composition pharmaceutique telle que revendiquée dans la revendication 7 sous une forme convenant à l'administration orale.

9. Composition pharmaceutique telle que revendiquée dans la revendication 8 pourvue d'un enrobage entérique.

10. Composé tel que revendiqué dans l'une quelconque des revendications 1 à 5 pour application comme agent thérapeutique.

11. Composé tel que revendiqué dans l'une quelconque des revendications 1 à 5 pour application dans le traitement de maladies gastro-intestinales causées ou exacerbées par l'acidité gastrique.

12. Procédé pour la préparation d'un composé tel que revendiqué dans la revendication 1, qui comprend
(a) la réaction d'un composé de structure (II)

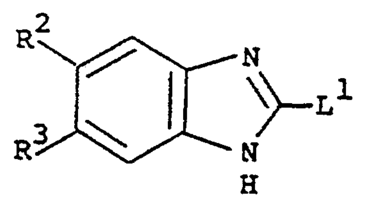

(II)

avec un composé de structure (III)

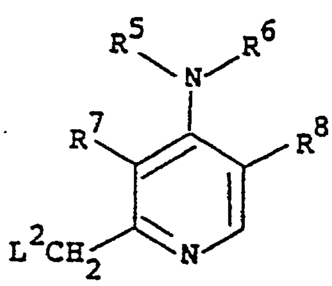

(III)

dans laqulle $R^2$, $R^3$ et $R^5$ à $R^8$ sont tels que décrits pour la structure (I), un des $L^1$ et $L^2$ est SH et l'autre est un groupement labile déplaçable par un mercaptan;
(b) la réaction d'un composé de structure (IV)

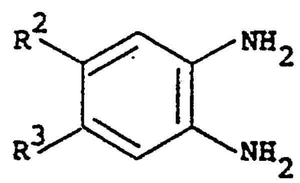

(IV)

dans laquelle $R^2$ et $R^3$ sont tels que décrits pour la structure (I), avec un composé de structure (V)

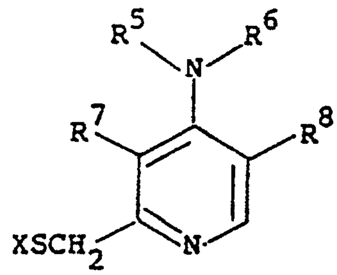

(V)

dans laquelle $R^5$ à $R^8$ sont tels que décrits pour la structure (I), X est $CO_2H$ ou $CSX^1$ et $X^1$ est un halogène ou un alkoxy en $C_{1-4}$;

(c) la réaction d'un composé de structure (VI)

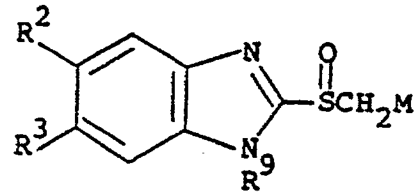

(VI)

dans laquelle $R^2$ et $R^3$ sont tels que décrits pour la structure (I) dans la revendication 1, $R^9$ est de l'hydrogène ou un groupement protecteur et M est un atome de métal alcalin, avec un composé de structure (VII)

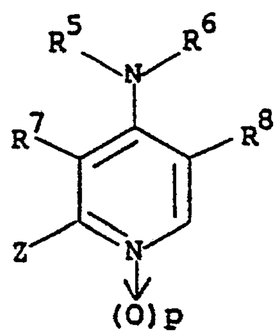

(VII)

dans laquelle $R^5$ à $R^8$ sont tels que décrit pour la structure (I), Z est un groupement labile et p est 0 ou 1;

(d) la réaction d'un composé de structure (VIII)

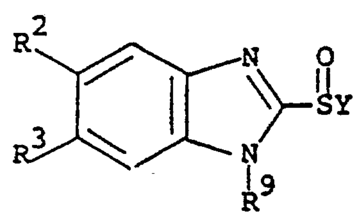

(VIII)

dans laquelle $R^2$ et $R^3$ sont tels que décrits pour la structure (I), $R^9$ est de l'hydrogène ou un groupement protecteur et Y est un groupement labile, avec un composé de structure (IX)

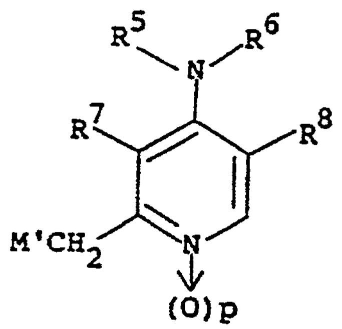

(IX)

dans laquelle $R^5$ à $R^8$ sont tels que décrits pour la structure (I), p est 0 ou 1 et M' est l'équivalent d'un atome de métal,
et, facultativement si on le désire,
(i) l'oxydation d'un composé de structure (I) dans laquelle n est 0 ainsi formé en un composé de structure (I) dans laquelle n est 1;
(ii) la réduction d'un composé de structure (I) ainsi formé dans laquelle n est 1 en un composé de structure (I) dans laquelle n est 0;
(iii) la formation d'un sel pharmaceutiquement acceptable.

13. Composé de structure (III)

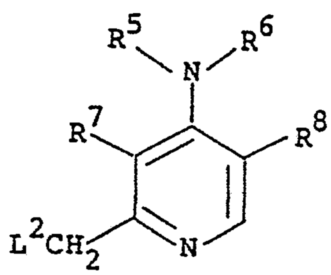

(III)

dans laquelle $R^5$ à $R^6$ sont tels que décrits pour la structure (I) et $L^2$ est SH ou un groupement labile déplaçable par un mercaptan.

14. Composé de structure (V)

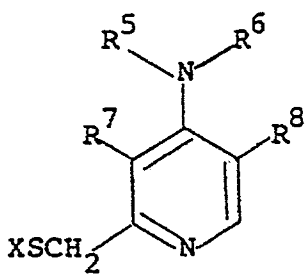

(V)

dans laquelle $R^5$ à $R^8$ sont tels que décrits pour la structure (I) et X est $CO_2H$ ou $CSX^1$ et $X^1$ est un halogène ou un alkoxy en $C_{1-4}$.

**Revendications pour l'Etat contractant AT:**

1. Procédé pour la préparation d'un composé de structure (I)

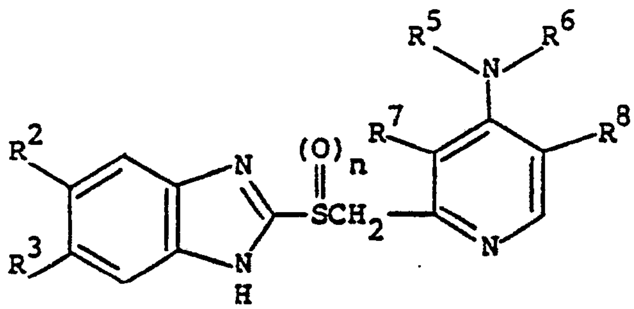

(I)

dans laquelle

$R^2$ et $R^3$ sont identiques ou différents et sont, chacun, de l'hydrogène, un alkyle en $C_{1-6}$, un alkoxy en $C_{1-6}$, $RCF_2O$, un groupement éthoxy substitué par 3 à 5 atomes de fluor ou $R^2$ et $R^3$ forment ensemble un groupement $-O(CR_2)_mO-$;

R est de l'hydrogène ou du fluor;

m est 1 ou 2;

n est 0 ou 1;

$R^5$ et $R^6$ sont identiques ou différents et sont chacun un alkyle en $C_{1-6}$ ou $R_5$ et $R_6$ forment ensemble avec l'atome d'azote auquel ils sont attachés un groupement pipéridino ou morpholino et un des $R^7$ et $R^8$ est un halogène et l'autre est de l'hydrogène, un halogène ou un alkyle en $C_{1-6}$;

ou un sel pharmaceutiquement acceptable de celui-ci qui comprend:

(a) la réaction d'un composé de structure (II)

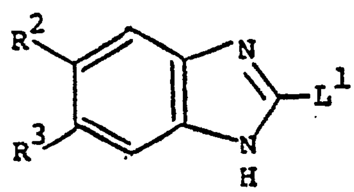

(II)

avec un composé de structure (III)

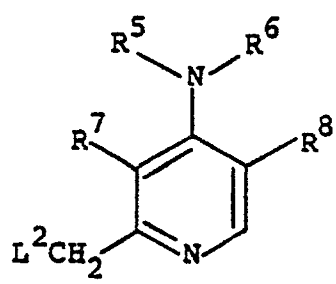

(III)

dans laquelle $R^2$, $R^3$ et $R^5$ à $R^8$ sont tels que décrits pour la structure (I), un des $L^1$ et $L^2$ est SH et l'autre est un groupement labile déplaçable par un mercaptan;

(b) la réaction d'un composé de structure (IV)

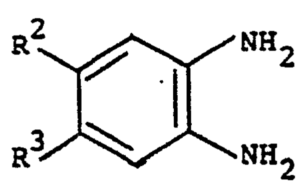

(IV)

dans laquelle $R^2$ et $R^3$ sont tels que décrits pour la structure (I), avec un composé de structure (V)

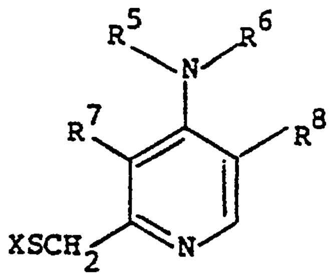

(V)

dans laquelle $R^5$ à $R^8$ sont tels que décrits pour la structure (I), X est $CO_2H$ ou $CSX^1$ et $X^1$ est un halogène ou un alkoxy en $C_{1-4}$.
(c) la réaction d'un composé de structure (VI)

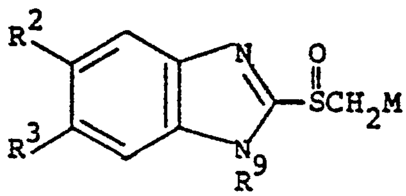

(VI)

dans laquelle $R^2$ et $R^3$ sont tels que décrits pour la structure (I), $R^9$ est de l'hydrogène ou un groupement protecteur et M est un atome de métal alcalin, avec un composé de structure (VII)

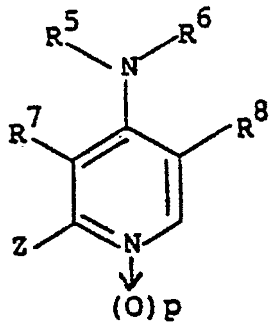

(VII)

dans laquelle $R^5$ à $R^8$ sont tels que décrits pour la structure (I), Z war un groupement labile et p est 0 ou 1
(d) la réaction d'un composé de structure (VIII)

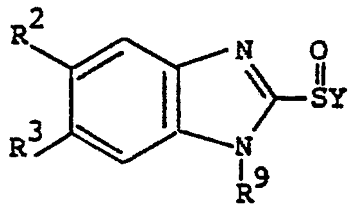

(VIII)

dans laquelle $R^2$ et $R^3$ sont tels que décrits pour la structure (I), $R^9$ est de l'ydrogène ou un groupement protecteur et Y est un groupement labile, avec un composé de structure (XI)

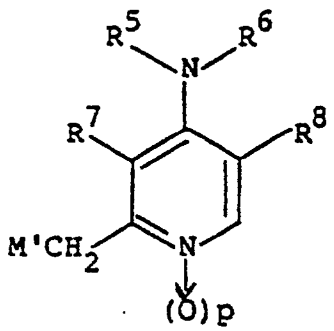

(IX)

dans laquelle $R^5$ à $R^8$ sont tels que décrits pour la structure (I), p est 0 ou 1 et M' est l'équivalent d'un atome de métal,

et, facultativement, si on le désire,

(i) l'oxydation d'un composé de structure (I) dans laquelle n est 0 ainsi formé en un composé de structure (I) dans laquelle n est 1;

(ii) la réduction d'un composé de structure (I) ainsi formé dans laquelle n est 1 en un composé de structure (I) dans laquelle n est 0;

(iii) la formation d'un sel pharmaceutiquement acceptable.

2. Procédé tel que revendiqué dans la revendication 1 dans lequel $L^1$ est SH et $L^2$ est un groupement labile.

3. Procédé tel que revendiqué dans la revendication 2 dans lequel $L^2$ est du chlore.

4. Procédé tel que revendiqué dans l'une quelconque des revendications 1 à 3 lorsqu'il est utilisé pour préparer un composé tel que décrit dans la revendication 1 dans laquelle n est 1.

5. Procédé tel que revendiqué dans l'une quelconque des revendications 1 à 4 lorsqu'il est utilisé pour préparer un composé décrit dans la revendication 1 dans laquelle $R^5$ et $R^6$ forment ensemble avec l'atome d'azote auquel ils sont attachés un groupement pipéridino ou morpholino.

6. Procédé tel que revendiqué dans l'une quelconque des revendications 1 à 5 lorsqu'il est utilisé pour préparer les

2-(3-chloro-4-pipéridino-2-pyridylméthylsulfinyl)-5-méthoxy-(1H)-benzimidazole,

2-(3-chloro-5-méthyl-4-pipéridino-2-pyridylméthylsulfinyl)-5-méthoxy-(1H)-benzimidazole,

2-(3-chloro-4-morpholino-2-pyridylméthylsulfinyl)-5-méthoxy-(1H)-benzimidazole,

2-(3-chloro-5-méthyl-4-morpholino-2-pyridylméthylsulfinyl)-5-méthoxy-(1H)-benzimidazole,

ou un sel pharmaceutiquement acceptablre de ceux-ci.

7. Procédé tel que revendiqué dans l'une quelconque des revendications 1 à 5 lorsqu'il est utilisé pour préparer le 2-(3-méthyl-5-bromo-4-pipéridino-2-pyridylméthylsulfinyl)-5-méthoxy-(1H)-benzimidazole.

8. Procédé pour préparer une composition pharmaceutique qui comprend la mise en association d'un composé tel que décrit dans la revendication 1 avec un support pharmaceutiquement acceptable.

9. Procédé tel que revendiqué dans la revendication 8 dans lequel n est 1.

10. Procédé tel que revendiqué dans la revendication 9 dans lequel la composition est, de plus pourvue d'un enrobage entérique.